# EUROPEAN PATENT APPLICATION

(11) **EP 4 527 392 A1**
(43) Date of publication of application: **26.03.2025**
(21) Application number: 23806929.8
(22) Date of filing: 16.05.2023
(51) Int. Cl.: A61K 31/737, A61K 47/18, A61P 3/06, A61P 29/00, A61P 19/02, A61P 39/06, A61P 43/00

(54) **CHONDROITIN SULFATE BIOLOGICAL POLYAMINE COMPOUND, PREPARATION METHOD THEREFOR AND USE THEREOF**

(30) Priority: 18.05.2022 CN 202210551251
(71) Applicant: Zhu, Xiaofeng, Loudi, Hunan 417700 (CN)
(72) Inventor: ZHAO, Yuxiang, Hunan 417700 (CN); GAO, Fanbo, Hunan 417700 (CN); SONG, Fengjie, Hunan 417700 (CN); MA, Chenyang, Hunan 417700 (CN); ZHU, Xingyu, Hunan 417700 (CN); ZHU, Xiaofeng, Hunan 417700 (CN)
(74) Representative: Perani & Partners S.p.A.
(86) International application number: PCT/CN2023/094524
(87) International publication number: WO 2023/221977

(57) **Abstract**

Disclosed in the present invention are a chondroitin sulfate biological polyamine compound, a preparation method therefor and use thereof. The present invention provides a chondroitin sulfate biological polyamine compound, which is a compound of chondroitin sulfate and biological polyamine. The chondroitin sulfate is non-covalently linked to the biological polyamine, and the biological polyamine comprises one, two, three or more of combinations of spermine, spermidine, putrescine and cadaverine. The chondroitin sulfate biological polyamine compound provided by the present invention has the anti-inflammatory activity remarkably superior to that of common chondroitin sulfate sodium, can be used in the prevention and treatment of inflammatory diseases, especially arthritis and joint damage, as well as the repair of bone tissues, and has the effects of reducing blood fat, anti-oxidation, slowing down aging, prolonging life span and the like.

## Description

### PRIORITY RIGHT AND RELATED APPLICATION

The present application claims the benefit of a priority of Chinese Patent Application No. 202210551251.3, filed on May 18, 2022 and entitled "CHONDROITIN SULFATE BIOLOGICAL POLYAMINE COMPOUND, PREPARATION METHOD THEREFOR AND USE THEREOF", the entire contents of which including the appendices are incorporated herein by reference.

### TECHNICAL FIELD

The present disclosure pertains to the field of natural medicine, and specifically relates to extraction of chondroitin sulfate, and to a chondroitin sulfate biogenic polyamine complex, a preparation method therefor, and use thereof.

### BACKGROUND

Chondroitin Sulfate (CS), a major member of the proteoglycan family, is a representative of animal mucopolysaccharides. It is ubiquitously present in a variety of animal tissue, particularly rich in cartilage and connective tissue. Chondroitin sulfate is not only a raw material for pharmaceuticals but also a raw material for health foods and cosmetics. It is extracted from cartilage tissue of animals, and has abundant sources and diverse structures. Chondroitin sulfate is useful for treatment of arthritis, neuralgia, neuropathic migraine, etc., and has an adjuvant therapeutic effect on chronic nephritis, chronic hepatitis, keratitis, corneal ulcer, etc. There are also some research reports on the prevention and treatment of such disease as coronary heart disease, angina pectoris, and myocardial infarction. Chondroitin sulfate has some effect on alleviation of the pain and symptoms caused by arthritis and inhibition of the progression of diseases.

However, albeit many clinical studies on chondroitin sulfate around the world, it is rarely used as a prescription drug for treatment of bone tissue-associated diseases. This is because it is not very effective clinically and administered at a high dose, and is even found to be virtually ineffective in some clinical studies (Cited Literature 3). The major reason for this controversy consists in the differences in the purity, type, and experimental population of chondroitin, and the limited bioactivity of chondroitin sulfate *per se,* so it is not enough to show stable and reliable significant differences. Meanwhile, chondroitin sulfate as a biologically derived polysaccharide has extremely high safety, and no obvious toxic and side effects have been found so far. However, most of other anti-inflammatory drugs generally have great side effects and are not suitable for long-term use. Therefore, how to substantially improve the bioactivity of chondroitin sulfate and develop new chondroitin sulfate substances with strong effectiveness and high safety is of great significance.

Researchers have afforded many efforts to improve the activity of chondroitin. It has been found in earlier reports that low-molecular-weight chondroitin sulfate could reduce viscosity, improve tissue permeability, and enhance bioactivity. Studies on low-molecular-weight polysaccharides such as low-molecular-weight chondroitin sulfate have also been reported recently. Of these, Cited Literature 1 discloses a method for preparing low-molecular-weight polysaccharide and a catalyst used therein, which produces a low-molecular-weight chondroitin product by adopting a polysaccharide degradation method using a metal solid-phase catalyst. Cited Literature 2 discloses use of low-molecular-weight chondroitin sulfate in the preparation of an external preparation for acne removal. However, the low-molecular-weight chondroitin sulfate produces a limited improvement in the anti-inflammatory activity in general, and almost no low-molecular-weight chondroitin has been commercially available to date.

As for the preparation method of chondroitin sulfate, Cited Literature 3 mentions that the products obtained by the existing preparation methods of chondroitin sulfate or a mixture containing the same have problems of unstable quality and efficacy, and even have side effects for the main reasons of numerous biological sources of raw materials, variety of production processes, great differences in molecular structures, different molecular weights, etc.

Therefore, although researchers have made many attempts to improve the activity of chondroitin sulfate, stable and highly active chondroitin sulfate or complexes including the same and preparation methods thereof are still needed to be further studied.

In some reports such as Cited Literature 4 and Cited Literature 5, a combination of chondroitin sulfate and polyamine has also been used. Nonetheless, Cited Literature 4 only uses the combination of chondroitin sulfate and polyamine as a matrix, but fails to study the bioactivity of the complex itself (drug). Furthermore, polyamine accounts for a high proportion in its composition, thereby forming micron particles with poor stability. In contrast, the present application produces a water-soluble complex with good stability. In addition, although Cited Literature 5 discloses a supramolecular complex of a polyanionic polymer and spermidine, it has not studied the effects of the molecular weight distribution of chondroitin, the type of polyamine, the protein content, and the like on the activity of the complex.

### Cited Literatures

Cited Literature 1: CN111495428A
Cited Literature 2: CN111110695A
Cited Literature 3: Discrepancies in Composition and Biological Effects of Different Formulations of Chondroitin Sulfate. Molecules 2015, 20, 4277-4289
Cited Literature 4: Poly-ion Complex of Chondroitin Sulfate and Spermine and Its Effect on Oral Chondroitin Sulfate Bioavailability. Chem. Pharm. Bull. 2016, 64, 390-398
Cited Literature 5: CN105797159A

### SUMMARY

### Technical Problem

As described above, in order to improve the pharmacological activity of chondroitin sulfate, researchers have made many attempts. According to earlier reports, the low-molecular-weight chondroitin sulfate could reduce viscosity, improve tissue permeability, and enhance bioactivity, but it produces a limited improvement in the anti-inflammatory activity in general, and almost no low-molecular-weight chondroitin has been commercially available to date. Meanwhile, there are also no methods for extracting or preparing stable and highly active chondroitin sulfate or a complex containing the same.

The inventors' research has found that due to the differences in extraction conditions, processes, etc., the extracted chondroitin varies greatly in activity, and some of the chondroitin exhibit a significantly better anti-inflammatory activity than those of the others. After a further study on their structures, the present disclosure confirms the structure and composition of this kind of chondroitin, and proves that it is the chondroitin sulfate biogenic polyamine complex provided herein which has a much better anti-inflammatory activity than that of ordinary chondroitin.

Therefore, in order to resolve the above problem, the present disclosure provides a chondroitin sulfate biogenic polyamine complex having an ultrahigh activity and a preparation method therefor. The chondroitin sulfate biogenic polyamine complex could substantially improve the anti-inflammatory (especially against arthritis) activity of chondroitin sulfate, and is expected to make up for the vacancy of clinical treatment of osteoarthritis. In the meantime, it has also been found to exhibit the efficacies of lowering blood lipids, anti-oxidation, delaying aging, extending lifespan, etc. There are currently no reports on the super strong anti-inflammatory activity of the chondroitin sulfate biogenic polyamine complex as well as its efficacies of repairing joint injuries, lowering blood lipids, anti-oxidation, delaying aging, extending lifespan, etc.

### Solution to Problem

In the first aspect of the present disclosure, there is provided a chondroitin sulfate biogenic polyamine complex, which is a complex of chondroitin sulfate and biogenic polyamine, wherein the chondroitin sulfate is non-covalently linked to the biogenic polyamine, and the biogenic polyamine comprises one or a combination of two or a combination of three or more of spermine, spermidine, putrescine, and cadaverine.

In some embodiments, the chondroitin sulfate is chondroitin sulfate in an acid form or chondroitin sulfate in a salt form.

In some embodiments, among the chondroitin sulfate, according to a GPC integral ratio, a proportion of chondroitin sulfate having a weight-average molecular weight of 50000 or more is 0%.

In some embodiments, among the chondroitin sulfate, according to the GPC integral ratio, a proportion of chondroitin sulfate having a weight-average molecular weight of 25000 to 50000 is 40% or less.

In some preferred embodiments, among the chondroitin sulfate, according to the GPC integral ratio, the proportion of the chondroitin sulfate having a weight-average molecular weight of 25000 to 50000 is 35% or less.

In some more preferred embodiments, among the chondroitin sulfate, according to the GPC integral ratio, the proportion of the chondroitin sulfate having a weight-average molecular weight of 25000 to 50000 is 30% or less, 29% or less, 28% or less, 27% or less, 26% or less, 25% or less, 24% or less, 23% or less, 22% or less, 21% or less, 20% or less, 19% or less, 18% or less, 17% or less, 16% or less, 15% or less, 14% or less, 13% or less, 12% or less, 11% or less, 10% or less, 9% or less, 8% or less, 7% or less, 6% or less, 5% or less, 4% or less, 3% or less, 2% or less, 1% or less, or 0%.

In some embodiments, among the chondroitin sulfate, according to the GPC integral ratio, an upper limit of a proportion of chondroitin sulfate having a weight-average molecular weight of 400 to 25000 is 80% or more, preferably 81%, 82%, 83%, 84%, 85%, 86%, 87%, 88%, 89%, 90%, 91%, 92%, 93%, 94%, 95%, 96%, 97%, 98%, 99% or more, or 100%; and according to the GPC integral ratio, a lower limit of the proportion of chondroitin sulfate having a weight-average molecular weight of 400 to 25000 is 40% or more, preferably 41%, 42%, 43%, 44%, 45%, 46%, 47%, 48%, 49%, 50%, 51%, 52%, 53%, 54%, 55%, 56%, 57%, 58%, 59% or 60% or more.

In some embodiments, among the chondroitin sulfate, according to the GPC integral ratio, a proportion of chondroitin sulfate having a weight-average molecular weight of 400 or less is 15% or less, preferably 3% or less, more preferably 1% or less, most preferably 0%.

In some embodiments, a proportion of chondroitin sulfate having an upper limit of the weight-average molecular weight of 25000 or less, preferably 24000, 23000, 22000, 21000, 20000, 19000, 18000, 17000, 16000, 15000, 14000, 13000, 12000, 11000, 10000, 9000, or 8000 or less and a lower limit of the weight-average molecular weight of 400, 500, 600, 700, or 800 or more has an upper limit of 80% or more, preferably 81%, 82%, 83%, 84%, 85%, 86%, 87%, 88%, 89%, 90%, 91%, 92%, 93%, 94%, 95%, 96%, 97%, 98%, 99% or more, or 100%, and has a lower limit of 40% or more, preferably 41%, 42%, 43%, 44%, 45%, 46%, 47%, 48%, 49%, 50%, 51%, 52%, 53%, 54%, 55%, 56%, 57%, 58%, 59%, or 60% or more.

In some embodiments, among the chondroitin sulfate, according to the GPC integral ratio, a proportion of chondroitin sulfate having a weight-average molecular weight of 400 to 10000 is 40% to 100%, preferably 50% to 100%, 60% to 100%, 70% to 100%, or 80% to 100%.

In some further preferred embodiments, according to the GPC integral ratio, the chondroitin sulfate has the following molecular weight distribution, in which the proportion of chondroitin sulfate having a weight-average molecular weight of greater than 50000 is 0%, the proportion of chondroitin sulfate having a weight-average molecular weight of 25000 to 50000 is 0% to 40%, the proportion of chondroitin sulfate having a weight-average molecular weight of 400 to 10000 and preferably 400 to 8000 is 40% to 100%, and the proportion of chondroitin sulfate having a weight-average molecular weight of 400 or less is 15% or less.

In some embodiments, the chondroitin sulfate biogenic polyamine complex has a weight-average molecular weight of 400 to 50000.

In some preferred embodiments, the chondroitin sulfate biogenic polyamine complex has a weight-average molecular weight of 400 to 25000.

In all embodiments, a sum of the proportion of the chondroitin sulfate having a weight-average molecular weight of 25000 to 50000, the proportion of the chondroitin sulfate having a weight-average molecular weight of 400 to 25000, and the proportion of the chondroitin sulfate having a weight-average molecular weight of 400 or less is 100%.

In some embodiments, based on a total weight (g) of the chondroitin sulfate biogenic polyamine complex, the chondroitin sulfate biogenic polyamine complex contains 200 µmol/g or less, preferably 199 µmol/g or less, 198 µmol/g or less, 197 µmol/g or less, 196 µmol/g or less, 195 µmol/g or less, 194 µmol/g or less, 193 µmol/g or less, 192 µmol/g or less, 191 µmol/g or less, 190 µmol/g or less, 189 µmol/g or less, 188 µmol/g or less, 187 µmol/g or less, 186 µmol/g or less, 185 µmol/g or less, 184 µmol/g or less, 183 µmol/g or less, 182 µmol/g or less, 181 µmol/g or less, or 180 µmol/g or less of the biogenic polyamine; and the chondroitin sulfate biogenic polyamine complex contains 0.5 µmol/g or more, preferably 0.6 µmol/g or more, 0.7 µmol/g or more, 0.8 µmol/g or more, 0.9 µmol/g or more, or 1 µmol/g or more of the biogenic polyamine.

In some embodiments, a mass percentage of protein in the chondroitin sulfate biogenic polyamine complex is less than 8%, preferably less than 5%, more preferably less than 3%, most preferably less than 1%, and further preferably 0%.

In the second aspect of the present disclosure, there is provided a preparation method of the chondroitin sulfate biogenic polyamine complex according to the first aspect, wherein the preparation method comprises a step of mixing chondroitin sulfate and biogenic polyamine, wherein based on a total weight (g) of the chondroitin sulfate biogenic polyamine complex, the chondroitin sulfate biogenic polyamine complex contains 0.5 to 200 µmol/g of the biogenic polyamine.

In the third aspect of the present disclosure, there is provided a preparation method for the chondroitin sulfate biogenic polyamine complex according to the first aspect, wherein the preparation method comprises a step of mixing an extraction solution containing chondroitin sulfate and polyamine separated and extracted from a raw material with ethanol, wherein the extraction solution has a pH value of 4 to 6, and a volume ratio of the extraction solution to ethanol is 1:1 to 1:3.

In some embodiments, separating and extracting the chondroitin sulfate and the polyamine from the raw material comprises:
a step of enzymolysis or acidolysis, wherein the raw material is lysed by enzymolysis or acidolysis to obtain an enzymatic hydrolyzate or an acid hydrolysate; and
a step of separating and extracting the chondroitin sulfate and the polyamine, wherein the chondroitin sulfate are extracted and the polyamine simultaneously or stepwise from the enzymatic hydrolysate or the acid hydrolysate.

In some embodiments, for stepwise extraction of the chondroitin sulfate and the polyamine, the polyamine is separated from the enzymatic hydrolysate or the acid hydrolysate by chromatography or extraction, and after the polyamine is separated, a residue is treated by one or more of enzymolysis, protein precipitation method, chromatography, and alcohol precipitation method to separate the chondroitin sulfate from the residue; alternatively, a step of depolymerizing the chondroitin sulfate is further included after the chondroitin sulfate is separated.

In some embodiments, for simultaneous extraction of the chondroitin sulfate and the polyamine, a protein precipitation method is adopted to precipitate a protein in the enzymatic hydrolysate or the acid hydrolysate so as to separate the chondroitin sulfate and the polyamine.

In some embodiments, the raw material comprises an animal tissue, a plant tissue, and a microbial culture fermentation broth.

In the fourth aspect of the present disclosure, there is provided a chondroitin sulfate biogenic polyamine complex prepared and obtained by the preparation method according to the second or third aspect of the present disclosure.

In the fifth aspect of the present disclosure, there is provided use of a chondroitin sulfate biogenic polyamine complex, wherein the use is any one of the following (a) to (g):
(a) use in the preparation of a drug for anti-inflammation;
(b) use in the preparation of a drug for treating and/or preventing an inflammatory disease;
(c) use in the preparation of a drug for lowering blood lipids;
(d) use in the preparation of a drug for treating and/or preventing hyperlipidemia;
(e) use in the preparation of a drug for treating and/or repairing a joint injury;
(f) use in the preparation of a drug for anti-oxidation; and
(g) use in the preparation of a drug for delaying aging and/or extending a lifespan;
   wherein the chondroitin sulfate biogenic polyamine complex is the chondroitin sulfate biogenic polyamine complex according to the first or fourth aspect of the present disclosure and/or a chondroitin sulfate biogenic polyamine complex prepared and obtained by the preparation method according to the second or third aspect of the present disclosure.

In some embodiments, wherein the inflammatory disease comprises inflammation caused by an inflammation-inducing factor and/or induced by an inflammatory cell, interleukin and/or a tumor necrosis factor, preferably, the inflammatory disease is one or more selected from allergy, eczema, myocardial infarction, cerebral infarction, Alzheimer's disease, dermatitis or arthritis.

In some embodiments, the hyperlipidemia comprises primary hyperlipidemia and/or secondary hyperlipidemia.

In some embodiments, the hyperlipidemia comprises hypertriglyceridemia and/or hypercholesterolemia.

In some embodiments, the hyperlipidemia comprises a hyperlipidemia-associated condition, alternatively, the hyperlipidemia-associated condition comprises a cardiovascular disease, optionally, the cardiovascular disease comprises one or more of arteriosclerosis, coronary artery disease, angina pectoris, carotid artery disease, stroke, cerebral arteriosclerosis, myocardial infarction, cerebral infarction, restenosis after balloon angioplasty, hypertension, intermittent claudication, dyslipidemia, postprandial lipemia, and xanthoma.

In some embodiments, the joint injury comprises a joint injury caused by inflammation, aging, exercise, or injury.

In the sixth aspect of the present disclosure, there is provided use of the chondroitin sulfate biogenic polyamine complex according to the first or fourth aspect of the present disclosure and/or a chondroitin sulfate biogenic polyamine complex prepared and obtained by the preparation method according to the second or third aspect of the present disclosure in the preparation of a health food or a cosmetic.

In the seventh aspect of the present disclosure, there is provided a pharmaceutical composition, health product or cosmetic, comprising the chondroitin sulfate biogenic polyamine complex according to the first or fourth aspect of the present disclosure and/or a chondroitin sulfate biogenic polyamine complex prepared and obtained by the preparation method according to the second or third aspect of the present disclosure.

### Advantageous Effects of the Invention

The chondroitin sulfate biogenic polyamine complex provided herein has a significantly better anti-inflammatory activity than that of ordinary sodium chondroitin sulfate, is useful for prevention and treatment of inflammatory diseases (especially arthritis and joint injury) and for bone tissue repair, and has the efficacies of lowering blood lipids, anti-oxidation, delaying aging, extending lifespan, etc.

### BRIEF DESCRIPTION OF THE DRAWINGS

FIG. 1A is a schematic diagram for the test results of IL-6 in serum of mice in different groups in Test Example 1.
FIG. 1B is a schematic diagram for the test results of IL-1β in serum of mice in different groups in Test Example 1.
FIG. 2A is a schematic diagram for the changing curves of degrees of swelling of mouse toes in different groups in Test Example 1.
FIG. 2B shows photographs of the toes and anatomical photographs of mice in different groups in Test Example 1. The arrows indicate the sites where the apparent inflammation occurs.
FIG. 3A is a schematic diagram for thickness data on rat toes in different groups in Test Example 2.
FIG. 3B shows photographs of the rat toes in different groups in Test Example 2. The arrows indicate the sites where the apparent inflammation occurs.
FIG. 4A to FIG. 4E are schematic diagrams for the results of blood routine analysis of rats in different groups in Test Example 2. FIG. 4A shows the absolute lymphocyte count; FIG. 4B shows the lymphocytes percentage; FIG. 4C shows the absolute neutrophil count; FIG. 4D shows the neutrophils percentage; and FIG. 4E shows the absolute leukocyte count.
FIG. 5A to FIG. 5C are schematic diagrams for the determination results of the organ indices of rats in different groups in Test Example 2. FIG. 5A shows the liver index; FIG. 5B shows the spleen index; and FIG. 5C shows the thymus index.
FIG. 6 shows CT photographs of the ankle joints of rats in different groups in Test Example 2.
FIG. 7 shows partial enlarged CT images of the ankle joints of rats in different groups in Test Example 2. The arrows indicate some of joint junctions in the model group.
FIG. 8A to FIG. 8F are schematic diagrams for the CT results of rats in different groups in Test Example 2. FIG. 8A shows the bone mineral density; FIG. 8B shows the bone surface; FIG. 8C shows the bone volume; FIG. 8D shows the ratio of the bone surface to the bone volume; FIG. 8E shows the trabecular separation/spacing; and FIG. 8F shows the trabecular thickness.
FIG. 9A to FIG. 9D are schematic diagrams for the blood lipid test results of rats in different groups in Test Example 3. FIG. 9A shows the level of serum total cholesterol in mice; FIG. 9B shows the level of serum total triglycerides in mice; FIG. 9C shows the level of serum low density lipoproteins in mice; and FIG. 9D shows the level of serum high density lipoproteins in mice.
FIG. 10 is a schematic diagram for the number of reactive oxygen species-positive cells in Test Example 5, with the control group on the left and the CSX-administered groups on the right.
FIG. 11 is a schematic diagram for the test result of the CSX activity against inflammation associated with Alzheimer's disease in Test Example 6.
FIG. 12A to FIG. 12C are schematic diagrams for the effects of CSX on inhibition of the inflammatory response at the cellular level in Test Example 7; FIG. 12A shows IL-6; FIG. 12B shows IL-1β; and FIG. 12C shows TNF-α.
FIG. 13 is a schematic diagram for result of the delaying aging-delay/lifespan-extension activity of CSX in Test Example 8.
FIG. 14 is a schematic diagram for inhibitory effects of the extracts and existing chondroitin on IL-6 in Example 1.
FIG. 15 is an H NMR spectrum of the extract in Example 1.
FIG. 16 is a partial enlarged image of H NMR spectra of chondroitin in different extracts in Example 1 (chemical shift: 3.0 ppm).
FIG. 17 is a chromatogram showing separation of the clear solution containing a polyamine component in the extract of Example 1.
FIG. 18 is a schematic diagram for inhibitory effects of various substances on IL-6 in Example 1.
FIG. 19 is a schematic diagram for the binding force between chondroitin molecules and polyamine molecules in Example 1.

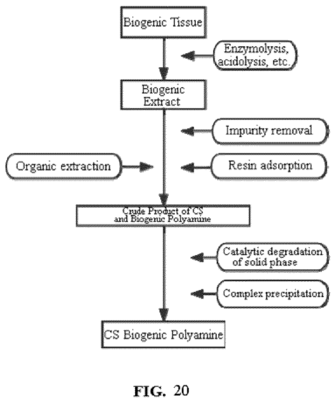

FIG. 20 is a flowchart diagram for the preparation method of a chondroitin sulfate biogenic polyamine complex according to the present disclosure.
FIG. 21A and FIG. 21B are schematic diagrams for result of the CSX activity in delaying aging/extending lifespan of *Drosophila melanogaster* in Test Example 8.
FIG. 22 is a schematic diagram for inhibitory effects of various substances on IL-6 in Test Example 9.
FIG. 23 is a schematic diagram for inhibitory effects of various substances on IL-6 in Test Example 10.
FIG. 24 is a schematic diagram for inhibitory effects of various substances on IL-6 in Test Example 12.
FIG. 25 is a schematic diagram for inhibitory effects of various substances on IL-6 in Test Example 13.

### DETAILED DESCRIPTION

The contents of the present disclosure will be described in detail below. The technical features described below are illustrated based on the representative embodiments or specific examples of the present disclosure, but the present disclosure is not limited thereto. The following should be noted:
The numerical range represented by "numerical value A to numerical value B" used in the present specification refers to the range including the endpoint values A and B.

The term "basically" or "substantially" used in the present specification means that the standard deviation from a theoretical model or theoretical data is within a range of 5%, preferably 3%, more preferably 1%.

The term "may" used in the present specification involves both the meaning of doing something and the meaning of not doing something.

In the present specification, the term "optional" or "optionally" means that the event or situation described subsequently may or may not occur, and the description includes the situation where the event occurs and the situation where the event does not occur.

In the present specification, the term "some specific/preferred embodiments", "other specific/preferred embodiments", "embodiment" or the like referred to means that the particular elements (*e.g*., features, structures, properties and/or characteristics) described in connection with this embodiment are included in at least one of the embodiments described herein, and may or may not exist in the other embodiments. In addition, it should be appreciated that the elements may be combined in any suitable manner in various embodiments.

The terms "comprising" and "having" and any of their variations used herein are intended to cover non-exclusive inclusions. For example, a process or method comprising a series of steps, a device, a product or equipment is not limited to the recited steps or modules, but optionally may further include unrecited steps, or optionally may further include other steps inherent to the process, method, product or equipment.

The term "a plurality of" referred to herein refers to two or more. The term "and/or" represents an association relationship for describing associated objects, and represents three possible relationships. For example, A and/or B may represent the following three cases: A exists alone, both A and B exist, and B exists alone. The character "/" generally indicates an alternative ("or") relationship between the associated objects.

In the present disclosure, the term "weight-average molecular weight (Mw)" is a relative molecular weight, a statistical average molecular weight by mass, or a molecular weight averaged over a unit weight, which can be determined, for example, by a light scattering method and gel chromatography. In some embodiments, the weight-average molecular weight is determined by high performance gel permeation chromatography, and the standard substance is a dextran molecular weight standards kit (National Institutes for Food and Drug Control) for determining the weight-average molecular weight of chondroitin sulfate/chondroitin sulfate polyamine.

In the present disclosure, the term "chondroitin sulfate" (CS) refers to different sulfated glycosaminoglycans having different molecular weights, which are found in various animal tissue. The glycoskeleton of CS consists of repeating disaccharide units [4)-β-D-GlcA-(1→3)-β-D-GalNAc-(1→] linked by β-glycosidic bonds (1→4). In the present disclosure, the chondroitin sulfate includes polysaccharides in the acid form and in the salt-forming form.

CS is typically a mixture. CS extracted from terrestrial animals is substantially composed of chondroitin sulfate A (CSA) and chondroitin sulfate C (CSC). In addition, CS extracted from marine animals further includes chondroitin sulfate D (CSD), chondroitin sulfate E (CSE), and other types. In the present disclosure, CSA refers to CS characterized by consisting generally of CSA-type disaccharide units [4)-β-D-GlcA-(1→3)-β-D-GalNAc4SO₃--(1→] (the sulfate group is at the *O*-4 position of galactose). In the present disclosure, CSC refers to CS characterized by consisting generally of CSC-type disaccharide units [4)-β-D-GlcA-(1→3)-β-D-GalNAc6SO₃--(1→] (the sulfate group is at the *O*-6 position of galactose). CSD refers to CS characterized by consisting generally of CSD-type disaccharide units [4)-β-D-GlcA2SO₃-(1→3)-β-D-GalNAc6SO₃--(1→] (the sulfate groups are at the *O*-2 position of glucuronic acid and at the *O*-6 position of galactose). CSE refers to CS characterized by consisting generally of CSE-type disaccharide units [4)-β-D-GlcA-(1→3)-β-D-GalNAc4,6SO₃--(1→] (the sulfate group is at the *O*-4,6 position of galactose).

In the present disclosure, the "GPC integral ratio" means to leverage the Slice capability of the GPC software to view each molecular weight and the corresponding integral ratio in the chromatogram of the test sample, thereby obtaining the GPC integral ratio corresponding to each molecular weight range.

In the present disclosure, the term "biogenic amines (BAs)" is a collective term for a class of bioactive low-molecular-weight organic compounds containing amino groups. BA may be regarded as a substance generated by substituting alkyl or aryl for 1 to 3 hydrogen atoms in the ammonia molecule. It is an aliphatic or heterocyclic low-molecular-weight organic base, and is often found in animals, plants, and foods. According to the structure of biogenic amine, it can be divided into three categories: aliphatic amines such as putrescine, cadaverin, spermine, and spermidine; aromatic amines such as tyramine and phenylethylamine; and heterocyclic amines such as histamine and tryptamine. According to the composition of biogenic amine, it can also be divided into monoamines and polyamines (or referred as to biogenic polyamines), where monoamines include histamine, tyramine, tryptamine, phenylethylamine, etc., and polyamines include cadaverin, putrescine, spermine, and spermidine.

In the present disclosure, the term "prevention" or "treatment" refers to a reduction in the risk of acquiring or developing a disease or condition, *i.e.,* causing at least one of the clinical symptoms of the disease not to be developed in a subject who is susceptible to the disease or is not exposed to the pathogen prior to the onset of the disease. For example, treatment may comprise: (i) preventing a disease, disorder and/or condition from occurring in a patient who may be susceptible to but has not yet been diagnosed with the disease, disorder and/or condition; (ii) suppressing the disease, disorder and/or condition, *i.e.,* preventing its development; or (iii) alleviating the disease, disorder and/or condition, *i.e.,* causing regression of the disease, disorder and/or condition.

In the present disclosure, the term "effective amount" means an amount of a compound, when administered to a subject for treatment or prevention of a disease, that is sufficient to achieve such treatment or prevention. The "effective amount" may vary depending on the compound, the disease and its severity, the age and weight of the subject to be treated, etc. The "therapeutically effective amount" refers to an effective amount for therapeutic treatment. The "prophylactically effective amount" refers to an effective amount for prophylactic treatment.

In the present disclosure, the term "administration" refers to physical introduction of an agent into a subject by any of various methods and delivery systems known to a person skilled in the art. Exemplary routes of administration include intravenous, intramuscular, subcutaneous, intraperitoneal, spinal or other parenteral routes of administration, such as by injection or infusion.

In the present disclosure, the term "subject", "individual", and "patient" are well-known in the art, and are used interchangeably herein to refer to any subject (particularly a mammalian subject) in need of treatment. Examples include, but are not limited to, humans and other primates, including non-human primates such as chimpanzees and other ape and monkey species. The terms "individual", "subject", and "patient" do not themselves denote a particular age, sex, race, etc.

As used herein, the term "inflammatory disease" is a collective term for diseases in which inflammation serves as their primary destructive factor. Examples of inflammatory diseases include, but are not limited to, edema, dermitis (dermatitis), acne, oral ulcers (*e.g.,* inflammatory oral ulcers), allergies, atopy, asthma, conjunctivitis, periodontitis, rhinitis, otitis media, pharyngitis, tonsillitis, pneumonia, gastric ulcers, gastritis, Crohn's disease, colitis, hemorrhoids, gout, ankylosing spondylitis, rheumatic fever, systemic lupus erythematosus, fibromyalgia, psoriatic arthritis, osteoarthritis, rheumatoid arthritis, scapulohumeral periarthritis, tendinitis, tenosynovitis, myositis, hepatitis, cystitis, nephritis, Sjögren's syndrome, and multiple sclerosis. In the present disclosure, the "inflammatory disease" may also include diseases in which inflammation is involved (*e.g*., diseases belonging to inflammatory complications).

In the present disclosure, the term "chronic inflammatory disease" refers to a wide range of disorders and conditions characterized by the presence of chronic inflammation. Examples of the chronic inflammatory disease include dermatomyositis, Grave's disease, multiple sclerosis, myasthenia gravis, systemic lupus erythematosus (SLE), sarcoidosis, sicca syndrome, amyloidosis, Hashimoto thyroiditis, vasculitis, rheumatoid arthritis, reactive arthritis, polymyositis, scleroderma, Addison's disease, vitiligo, pernicious anemia, glomerulonephritis, severe celiac disease, type 1 diabetes, psoriasis, pulmonary fibrosis, and eczema. In the present disclosure, the "chronic inflammatory disease" may also include diseases in which chronic inflammation is involved (*e.g*., diseases belonging to chronic inflammatory complications). Examples of such "chronic inflammatory diseases" include myocardial infarction, cerebral necrosis, Alzheimer's disease, etc.

In the present disclosure, the term "arthritis" is a collective term for diseases associated with inflammatory changes that occur in the joint area as a result of bacterial infection or trauma. Arthritis is broadly divided into acute arthritis and chronic arthritis. Acute arthritis is further divided as follows: (1) Serous arthritis: It is usually induced by trauma, but it may also occur for unknown reasons. It usually occurs at one joint. (2) Serous fibrinous arthritis: It occurs with acute rheumatoid arthritis and causes accumulation of turbid exudate in the joint cavity. Even after the inflammation has subsided, it may cause dyskinesia due to the formation of pseudomembranes. (3) Septic arthritis: It is multiple arthritis that occurs in open wounds of the joints or in contagious diseases such as gonorrhea, typhoid fever, scarlet fever, and septicemia. Infants and children aged 1 to 2 months may develop joint dislocations due to severe non-healing damage to the bone. Adults often suffer from osteomyelitis in the periosteum, which causes rupture and allows pus to flow into joints, and is therefore known as secondary septic arthritis. Chronic arthritis can be further divided as follows: (1) Special types of inflammation: It generally refers to gouty arthritis caused by tuberculous arthritis or syphilitic arthritis or by metabolic disorders of uric acid common in middle-aged people. (2) Polyarthritis: Chronic rheumatoid arthritis is the most common. Acute serous arthritis may turn into polyarthritis, or it may occur as polyarthritis during pneumonia, syphilis, and gonorrhea, or it may be a type of septicemia. In addition, Still's disease also falls within this category. (3) Deformans arthritis: It is usually induced by degenerative aging processes or trauma. (4) Hemophilic arthritis: It is induced by joint bleeding in hemophilia patients. Degenerative arthritis, also known as osteoarthritis, is a localized arthritis caused by degenerative changes in articular cartilage and occurs mainly in middle-aged or elderly people.

In the present disclosure, the term "rheumatoid arthritis" is a chronic, systemic inflammatory disease that may infect many organs and whose cause remains unknown. This process produces, at the beginning, an inflammatory response to the synovial membrane surrounding the joint, which then gradually spreads to adjacent cartilage and bones, leading to destruction and deformation of the joint. Extra-articular clinical manifestations include anemia, sicca syndrome, subcutaneous nodules, pulmonary fibrosis, vasculitis, dermal ulcers, etc.

In the present disclosure, the term "hyperlipidemia" refers to a condition characterized by abnormal elevation of serum lipids. The lipid fraction in circulating blood includes, for example, total cholesterol, some lipoproteins, and triglycerides. Serum lipoproteins serve as carriers of lipids in the circulation and are classified according to their density into: chylomicrons, very low density lipoprotein (VLDL), intermediate density lipoprotein (IDL), low density lipoprotein (LDL), and high density lipoprotein (HDL). The term "hyperlipidemia" includes primary and secondary hyperlipidemia. Primary hyperlipidemia is typically caused by genetic defects, while secondary hyperlipidemia is typically caused by other factors, such as various morbid states, drugs, and dietary factors. For example, secondary hyperlipidemia may be caused by diabetes. Alternatively, hyperlipidemia may be caused jointly by primary and secondary factors. Hyperlipidemia may include hypertriglyceridemia, hypercholesterolemia or a combination thereof. The "hypertriglyceridemia" refers to a condition in which the level of serum total triglycerides is higher than the desired level. The "hypercholesterolemia" refers to a condition in which the level of serum cholesterol is higher than the desired level. In some embodiments, the level of serum total cholesterol, HDL cholesterol (HDL-C) or LDL cholesterol (LDL-C) in hypercholesterolemia is higher than the desired level. Hyperlipidemia also contributes to the risk of developing cardiovascular disease and atherosclerotic disease, and may promote the development of cardiovascular disease and atherosclerotic disease. The term "cardiovascular disease" includes vascular diseases of the circulatory system caused by an abnormally high level of lipids in blood vessels. The term "atherosclerosis" refers to a disease of the arteries in which fatty plaques form on the inner wall and ultimately block blood flow.

The term "pharmaceutically acceptable" (or "pharmacologically acceptable") refers to the molecular entities and compositions that do not produce adverse reactions, allergic reactions or other untoward reactions when administered to animals or humans, as appropriate. The term "pharmaceutically acceptable carrier" as used herein encompasses any and all solvents, dispersion media, coatings, antibacterial agents, isotonic agents, and absorption retarders, buffers, excipients, binders, lubricants, gels, surfactants, and the like, which may be used as media for pharmaceutically acceptable substances.

The present disclosure provides a chondroitin sulfate biogenic polyamine complex and a method for preparing a chondroitin sulfate biogenic polyamine complex from biological tissue. The chondroitin sulfate biogenic polyamine complex provided herein and a chondroitin sulfate biogenic polyamine complex (which may be abbreviated as "CSX" herein) obtained by the method of the present disclosure have a weight-average molecular weight of 400 to 50000. The chondroitin sulfate biogenic polyamine complexes have a significantly better anti-inflammatory activity than that of ordinary sodium chondroitin sulfate. The present disclosure further relates to use of a chondroitin sulfate biogenic polyamine complex obtained by the method of the present disclosure, which is useful for prevention and treatment of chronic inflammation, blood lipids lowering, in particular arthritis and joint injury, bone tissue repair, etc.

The chondroitin sulfate biogenic polyamine complex provided herein and the preparation method therefor and use thereof will be described in detail below.

### <Chondroitin Sulfate Biogenic Polyamine Complex>

In some aspects of the present disclosure, there is provided a chondroitin sulfate biogenic polyamine complex, which is a complex of chondroitin sulfate and biogenic polyamine, wherein the chondroitin sulfate is linked to the biogenic polyamine. In some specific embodiments, the chondroitin sulfate and the biogenic polyamine are linked via a non-covalent bond (non-covalent linkage). This complex functions as a drug itself, rather than as a matrix of an additional drug or active ingredient. The chondroitin sulfate biogenic polyamine complex provided herein is a natural drug, but it may also be synthesized by chemical methods.

In some embodiments, the chondroitin sulfate in the chondroitin sulfate biogenic polyamine complex includes one or a combination of two or more of chondroitin sulfate A, chondroitin sulfate C, chondroitin sulfate D, and chondroitin sulfate E. n represents a natural number of 1 to 100 or 90 or 80 or 70 or 60 or 50 or 40 or 30 or 20 or 16.

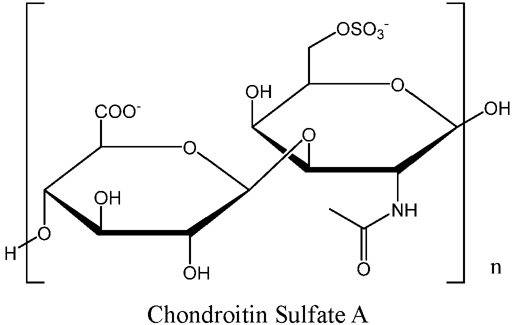

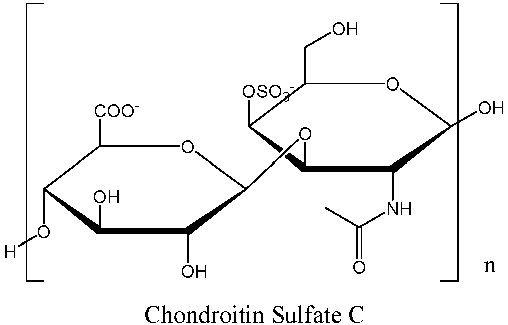

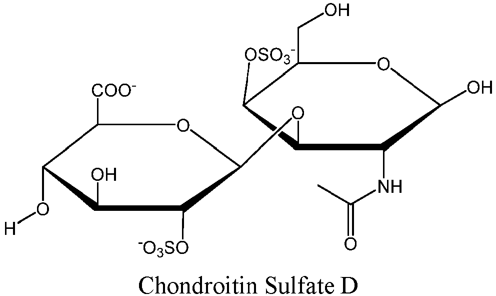

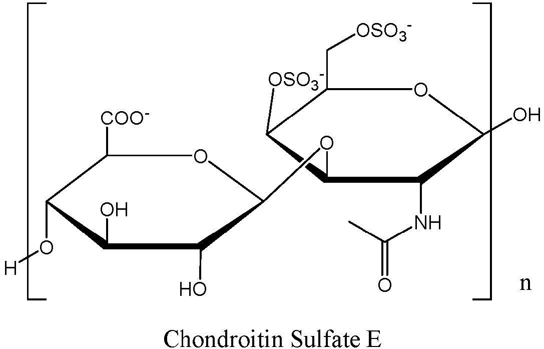

In some specific embodiments, according to the difference in chondroitin sulfate (*e.g.,* CSA, CSC, CSD, and CSE), the chondroitin sulfate biogenic polyamine complex may include a chondroitin sulfate biogenic polyamine complex having any one of the structures represented by the following Formula I (CSA), Formula II (CSC), Formula III (CSD), and Formula IV (CSE), or any combination thereof at any ratio.

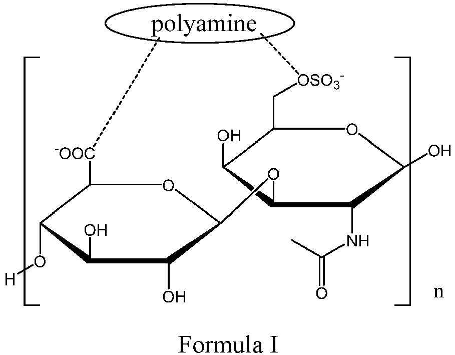

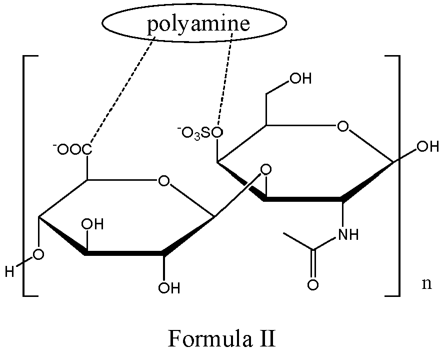

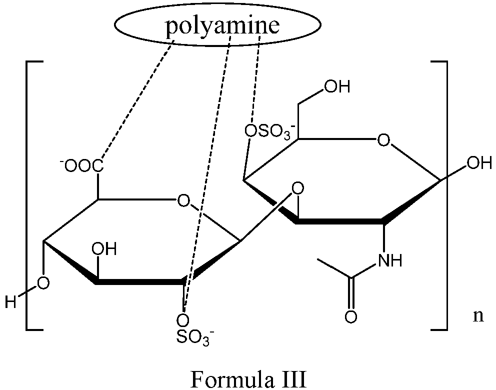

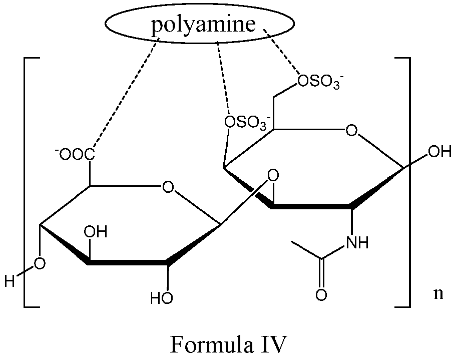

In Formula I to Formula IV, "polyamine" represents polyamine/biogenic polyamine. n represents a natural number of 1 to 100 or 90 or 80 or 70 or 60 or 50 or 40 or 30 or 20 or 16.

Since chondroitin sulfate exists typically as a mixture, it can be understood that the chondroitin sulfate biogenic polyamine complex provided herein, especially the chondroitin sulfate biogenic polyamine complex extracted and prepared from, for example, natural raw materials such as bone tissue, is usually a mixture of different chondroitin sulfates linked to different biogenic polyamines.

In some embodiments of the present disclosure, the biogenic polyamine includes one or a combination of two or a combination of three or more of spermine, spermidine, putrescine, and cadaverine.

In some specific embodiments of the present disclosure, the biogenic polyamine includes one of spermine, spermidine, putrescine, and cadaverin.

Exemplarily, the chondroitin sulfate biogenic polyamine complex containing one biogenic polyamine may be:
a complex of chondroitin sulfate and spermine;
a complex of chondroitin sulfate and spermidine;
a complex of chondroitin sulfate and putrescine; and
a complex of chondroitin sulfate and cadaverin.

In other more specific embodiments of the present disclosure, the biogenic polyamine includes at least two of spermine, spermidine, putrescine, and cadaverin.

In some more specific embodiments of the present disclosure, the biogenic polyamine is a combination of:
spermine and spermidine;
spermine and putrescine;
spermine and cadaverin;
spermidine and putrescine;
spermidine and cadaverin;
putrescine and cadaverin;
spermine, spermidine, and putrescine;
spermine, spermidine, and cadaverin;
spermine, putrescine, and cadaverin;
spermidine, putrescine, and cadaverin; or
spermine, spermidine, putrescine, and cadaverin.

As will be demonstrated in the subsequent examples and test examples of the present disclosure, the chondroitin sulfate biogenic polyamine complex containing two or more biogenic polyamines generally achieves better activities in all aspects, as compared to the chondroitin sulfate biogenic polyamine complex containing only a single type of biogenic polyamine. At the same time, the chondroitin sulfate biogenic polyamine complex containing one biogenic polyamine also exhibits a significantly better activity than that of the currently available chondroitin sulfate, and such an activity includes, but is not limited to, an anti-inflammatory (especially against arthritis) activity, blood lipids lowering, anti-oxidation, aging delay, lifespan extension, etc.

In some embodiments of the present disclosure, the chondroitin sulfate biogenic polyamine complex has a weight-average molecular weight of 400 to 50000. Preferably, the chondroitin sulfate biogenic polyamine complex has a weight-average molecular weight of 400 to 25000.

In some embodiments of the present disclosure, among the chondroitin sulfate, according to the GPC integral ratio, the proportion of chondroitin sulfate having a weight-average molecular weight of 50000 or more is 0%.

In some embodiments of the present disclosure, among the chondroitin sulfate, according to the GPC integral ratio, a proportion of chondroitin sulfate having a weight-average molecular weight of 25000 to 50000 is 40% or less.

In some preferred embodiments of the present disclosure, among the chondroitin sulfate, according to the GPC integral ratio, the proportion of chondroitin sulfate having a weight-average molecular weight of 25000 to 50000 is 35% or less.

In some further preferred embodiments of the present disclosure, among the chondroitin sulfate, according to the GPC integral ratio, the proportion of chondroitin sulfate having a weight-average molecular weight of 25000 to 50000 is 30% or less, 29% or less, 28% or less, 27% or less, 26% or less, 25% or less, 24% or less, 23% or less, 22% or less, 21% or less, 20% or less, 19% or less, 18% or less, 17% or less, 16% or less, 15% or less, 14% or less, 13% or less, 12% or less, 11% or less, 10% or less, 9% or less, 8% or less, 7% or less, 6% or less, 5% or less, 4% or less, 3% or less, 2% or less, 1% or less, or 0%.

In some embodiments of the present disclosure, among the chondroitin sulfate, according to the GPC integral ratio, an upper limit of a proportion of chondroitin sulfate having a weight-average molecular weight of 400 to 25000 is 80% or more. Preferably, among the chondroitin sulfate, according to the GPC integral ratio, the upper limit of the proportion of chondroitin sulfate having a weight-average molecular weight of 400 to 25000 is 81%, 82%, 83%, 84%, 85%, 86%, 87%, 88%, 89%, 90%, 91%, 92%, 93%, 94%, 95%, 96%, 97%, 98%, 99% or more, or 100%.

In some embodiments of the present disclosure, among the chondroitin sulfate, according to the GPC integral ratio, a lower limit of the proportion of chondroitin sulfate having a weight-average molecular weight of 400 to 25000 is 40% or more. Preferably, according to the GPC integral ratio, the lower limit of the proportion of chondroitin sulfate having a weight-average molecular weight of 400 to 25000 is 41%, 42%, 43%, 44%, 45%, 46%, 47%, 48%, 49%, 50%, 51%, 52%, 53%, 54%, 55%, 56%, 57%, 58%, 59%, or 60% or more.

In some embodiments of the present disclosure, among the chondroitin sulfate, according to the GPC integral ratio, a proportion of chondroitin sulfate having a weight-average molecular weight of 400 or less is 15% or less, preferably 3% or less, more preferably 1% or less, most preferably 0%.

The proportion of chondroitin sulfate having the upper limit of the weight-average molecular weight of 25000 or less, preferably 24000, 23000, 22000, 21000, 20000, 19000, 18000, 17000, 16000, 15000, 14000, 13000, 12000, 11000, 10000, 9000, or 8000 or less and the lower limit of the weight-average molecular weight of 400, 500, 600, 700, or 800 or more has an upper limit of 80% or more, preferably 81%, 82%, 83%, 84%, 85%, 86%, 87%, 88%, 89%, 90%, 91%, 92%, 93%, 94%, 95%, 96%, 97%, 98%, 99%, or more or 100%, and has a lower limit of 40% or more, preferably 41%, 42%, 43%, 44%, 45%, 46%, 47%, 48%, 49%, 50%, 51%, 52%, 53%, 54%, 55%, 56%, 57%, 58%, 59%, or 60% or more.

The sum of the proportion of chondroitin sulfate having a weight-average molecular weight of 25000 to 50000, the proportion of chondroitin sulfate having a weight-average molecular weight of 400 to 25000, and the proportion of chondroitin sulfate having a weight-average molecular weight of 400 or less is 100%.

In some preferred embodiments of the present disclosure, among the chondroitin sulfate, according to the GPC integral ratio, a proportion of chondroitin sulfate having a weight-average molecular weight of 400 to 10000 is 40% to 100%, preferably 50% to 100%, 60% to 100%, 70% to 100%, or 80% to 100%.

In some further preferred embodiments of the present disclosure, according to the GPC integral ratio, the chondroitin sulfate has the following molecular weight distribution, in which the proportion of chondroitin sulfate having a weight-average molecular weight of greater than 50000 is 0%, the proportion of chondroitin sulfate having a weight-average molecular weight of 25000 to 50000 is 0% to 40%, the proportion of chondroitin sulfate having a weight-average molecular weight of 400 to 10000 and preferably 400 to 8000 is 40% to 100%, and the proportion of chondroitin sulfate having a weight-average molecular weight of 400 or less is 15% or less.

As will be mentioned later and described in the examples, the chondroitin sulfate biogenic polyamine complex provided herein can be prepared from a natural raw material by co-precipitation of chondroitin sulfate and biogenic polyamine in the natural raw material to form a complex in which the both are non-covalently linked. Accordingly, it can be understood that the ratio between the chondroitin sulfate and the biogenic polyamine in the complex is the same or similar to their ratio in the natural raw material used, and the complex is always active at any ratio before the biogenic polyamine bound to the chondroitin sulfate reaches the saturation state.

In some embodiments, based on the total weight (g) of the chondroitin sulfate biogenic polyamine complex, the chondroitin sulfate biogenic polyamine complex contains 200 µmol/g or less; preferably 199 µmol/g or less, 198 µmol/g or less, 197 µmol/g or less, 196 µmol/g or less, 195 µmol/g or less, 194 µmol/g or less, 193 µmol/g or less, 192 µmol/g or less, 191 µmol/g or less, 190 µmol/g or less, 189 µmol/g or less, 188 µmol/g or less, 187 µmol/g or less, 186 µmol/g or less, 185 µmol/g or less, 184 µmol/g or less, 183 µmol/g or less, 182 µmol/g or less, 181 µmol/g or less, or 180 µmol/g or less of the biogenic polyamine.

In some preferred embodiments, based on the total weight (g) of the chondroitin sulfate biogenic polyamine complex, the chondroitin sulfate biogenic polyamine complex contains 0.5 µmol/g or more; preferably 0.6 µmol/g or more, 0.7 µmol/g or more, 0.8 µmol/g or more, 0.9 µmol/g or more, or 1 µmol/g or more of the biogenic polyamine.

In some more preferred embodiments, based on the total weight (g) of the chondroitin sulfate biogenic polyamine complex, the chondroitin sulfate biogenic polyamine complex contains 0.5 to 200 µmol/g, preferably 0.6 to 200 µmol/g, 0.7 to 200 µmol/g, 0.8 to 200 µmol/g, 0.9 to 200 µmol/g, more preferably 1 to 200 µmol/g, 1 to 195 µmol/g, 1 to 190 µmol/g, 1 to 185 µmol/g, or 1 to 180 µmol/g of the biogenic polyamine. In some preferred embodiments, the biogenic polyamine is one or a combination of two or a combination of three or more of spermine, spermidine, putrescine, and cadaverine at any ratio.

In some embodiments, the chondroitin sulfate is chondroitin sulfate in the acid form (chondroitin sulfate acid) or chondroitin sulfate in the salt form. In some preferred embodiments, the chondroitin sulfate is chondroitin sulfate in the acid form, which binds more tightly to the biogenic polyamine and can bind more polyamine molecules, so that the chondroitin sulfate biogenic polyamine complex has a better bioactivity.

In the present specification, the "low-molecular CS", "low-molecular chondroitin" or "low-molecular-weight chondroitin" refers to chondroitin sulfate having a weight-average molecular weight of 400 to 8000, which can be prepared by degrading chondroitin sulfate by means of enzymolysis, acidolysis, oxidative free radical degradation, radiation degradation or the like. The chondroitin sulfate biogenic polyamine complex containing low-molecular CS is referred to as low-molecular CSX or low-molecular chondroitin sulfate biogenic polyamine complex. In the present specification, the "ordinary chondroitin" or "ordinary CS" refers to commercially-available conventional chondroitin.

### <Method for Preparing Chondroitin Sulfate Biogenic Polyamine Complex and Chondroitin Sulfate Biogenic Polyamine obtained by the Preparation Method>

The present disclosure has discovered that chondroitin sulfate polyamine has significantly improved activities in many aspects as compared to chondroitin sulfate, and also discovered that the chondroitin sulfate polyamine can be extracted and prepared from a natural raw material. However, in order to assure the properties such as purity and color of chondroitin, the existing methods for extracting chondroitin sulfate generally comprise the steps such as purification by ion resin exchange, decolorization by hydrogen peroxide, at least twice alcohol precipitation-dissolution. These steps render the polyamine almost completely eliminated while improving the purity of chondroitin. None of them focuses on the complexation of chondroitin sulfate and biogenic polyamine and the efficacy of the complex. Moreover, the existing methods usually involve higher ionic strength, which has a greater impact on the non-covalent bond, and the biogenic polyamine complex of the present disclosure cannot be formed even in the presence of a small amount of polyamines.

In view of the above problem, in some aspect of the present disclosure, there is provided a preparation method of the above-mentioned chondroitin sulfate biogenic polyamine complex, comprising a step of mixing the extraction solution containing chondroitin sulfate and polyamine separated and extracted from the raw material with ethanol, wherein the pH value of the extraction solution is 4 to 6, and the volume ratio of the extraction solution to ethanol is 1:1 to 1:3.

In the preparation method of a chondroitin sulfate biogenic polyamine complex provided herein, the extraction solution containing chondroitin sulfate and polyamine separated and extracted from the raw material is mixed with ethanol under certain conditions, such that the chondroitin sulfate and the polyamine form a complex, *i.e*., the chondroitin sulfate biogenic polyamine complex, which has a significantly better anti-inflammatory activity than that of ordinary chondroitin sulfate and has the effects of repairing joint injuries, lowering blood lipids, anti-oxidation, delaying aging, extending lifespan, etc.

In some specific embodiments, the pH value of the extraction solution may be 4, 4.5, 5, 5.5 or 6. In some preferred embodiments, the pH value of the extraction solution is 5. Unlike the neutral pH value used for the extraction and precipitation of conventional chondroitin sulfate, the extraction solution at the above pH value is conducive to formation of the chondroitin sulfate biogenic polyamine complex, for example, it can increase the content of the biogenic polyamine that can be co-precipitated and complexed with chondroitin sulfate, thereby facilitating formation of the chondroitin sulfate biogenic polyamine complex.

In some specific embodiments, the volume ratio of the extraction solution to the ethanol is 1:1 to 1:2.5, *e.g.,* 1:1 or 1:2.5. In some preferred embodiments, the ethanol accounts for 60% to 70% by volume of the mixed solution of the extraction solution and ethanol. At this volume ratio, it is more suitable for formation of the chondroitin sulfate biogenic polyamine complex. At this volume ratio, unlike the methods intended to pursue high-purity chondroitin sulfate, the preparation method of the present disclosure can improve the polyamine content and could reduce protein impurities despite loss of part of chondroitin sulfate, and thus facilitate formation of the chondroitin sulfate biogenic polyamine complex, which is usually unavailable under the prior-art conditions.

In some specific embodiments, the ethanol is anhydrous ethanol.

### (Source of Raw Material)

In the present disclosure, the source of raw material is not particularly limited, and may be any raw material containing chondroitin sulfate and/or polyamine, *e.g*., an animal tissue, a plant tissue, and a microbial culture fermentation broth.

In some embodiments, the raw material may be connective tissue of animals, including cartilage, bones, tendon, sarcolemma, vascular walls, etc. The animals may be terrestrial animals, such as cattle, pigs, and chickens, or may be marine animals, such as a variety of fishes. It can be appreciated by a person skilled in the art that the above raw materials include chondroitin sulfate and polyamine components. In some embodiments, the raw material may further include raw materials containing the polyamine components, such as soybeans and wheat germ.

The types of chondroitin sulfate contained in the resulting chondroitin sulfate biogenic polyamine complex vary with the source of raw material. Typically, CS extracted from terrestrial (animal) sources is mainly a mixture of CSA and CSC, and CS extracted from marine (animal) sources is mainly a mixture of CSA, CSC, and CSD with a few further containing CSE. Furthermore, raw materials from different species of animals contain varied proportions of CS of different types. Therefore, in the chondroitin sulfate biogenic polyamine complex obtained by the preparation method of a chondroitin sulfate biogenic polyamine complex provided herein, chondroitin sulfate may be any one of CSA, CSC, CSD, and CSE, or any combination thereof at any ratio.

In some preferred embodiments, chicken bones may be selected as a raw material. Chicken bones generally contain a high level of biogenic polyamine, and are cheaper with stable supply and reliable quality, and ready availability in large quantities.

### (Pretreatment)

Before carrying out the method of the present disclosure, it is often necessary to carry out a pretreatment step, so that the raw material is applicable to the method of the present disclosure (*e.g*., enzymolysis, acidolysis, and separation and extraction). The pretreatment method is not particularly limited. A person skilled in the art may select an appropriate pretreatment method depending upon different raw materials. For example, for raw materials such as bones and cartilage, the pretreatment method may include boiling, impurity removal, washing, drying, grinding and pulverizing, and the like. For another example, for soybeans, the pretreatment method may include grinding and pulverizing.

### (Separation and Extraction of Chondroitin Sulfate and Polyamine from Raw Material)

Before being mixed with ethanol and complex precipitated into a chondroitin sulfate biogenic polyamine complex, the chondroitin sulfate and polyamine in the raw material can be separated and extracted to increase the proportions of the chondroitin sulfate and polyamine in the raw material and yield a chondroitin sulfate biogenic polyamine complex with a higher purity, reduce impurities such as inorganic salts and proteins, and minimize the influence of the impurities on the subsequent complex precipitation process.

In some embodiments of the present disclosure, when chondroitin sulfate and polyamine are separated and extracted from the raw material, the raw material is subjected to enzymolysis or acidolysis, by which the raw material is lysed to obtain an enzymatic hydrolysate or an acid hydrolysate, so that the components such as chondroitin sulfate and/or polyamines in the raw material are fully released.

Inadequate enzymolysis or acidolysis will affect the release of polyamines. Therefore, in some embodiments, the raw material is subjected to adequate enzymolysis or acidolysis. A person skilled in the art may select an appropriate amount of enzymolysis or acidolysis reagent to realize adequate enzymolysis or acidolysis of the raw material.

In some embodiments, for the enzymolysis, the selectable enzyme includes one or more of papain, alkaline protease, neutral protease, acid protease, pepsin, trypsin, chymotrypsin, bromelain, and ficin protease. In some preferred embodiments, at least 4000U, preferably 10000U or more, 16000U or more, 20000U or more, 30000U or more, 40000U or more, or 50000U or more of enzyme is added to each gram of pretreated sample. The lower the activity of enzyme, the less likely it is to thoroughly hydrolyze the protein. Consequently, it is impossible to release the polyamine molecules from the raw material. Moreover, during protein precipitation method by trichloroacetic acid, polyamines are prone to binding to larger proteins to form precipitates, thereby further reducing the extraction amount of polyamines and thus affecting the activity of the product. In some embodiments, the temperature of enzymolysis is from 50°C to 70°C, preferably from 55°C to 65°C, for example, 55°C, 60°C or 65°C. In some embodiments, the time of enzymolysis is 1 to 24 h, preferably 3 to 24 h, 3 to 16 h, for example, 3 h, 6 h or 16 h. In some embodiments, the enzymolysis may be carried out once. In other embodiments, the enzymolysis may be carried out twice, thrice or more times.

In some embodiments, an organic acid and/or inorganic acid may be used for acidolysis. In some embodiments, the organic acid may include one or more of trifluoroacetic acid, trichloroacetic acid, formic acid, and acetic acid. In some embodiments, the inorganic acid may include one or more of hydrochloric acid, sulfuric acid, and nitric acid. In some embodiments, the acidolysis may be carried out with the aid of the methods such as ultrasonic extraction, addition or grinding to improve the efficiency of acidolysis, fully lyse the raw material, and release the components such as chondroitin sulfate and/or polyamines.

In some embodiments of the present disclosure, chondroitin sulfate and polyamine are separated and extracted from the enzymatic hydrolysate or acid hydrolysate obtained after enzymolysis or acidolysis. In some embodiments, chondroitin sulfate and polyamine may be separated and extracted stepwise. The stepwise extraction can maximize the extraction amount of polyamine, improve the weight ratio of polyamine in the chondroitin sulfate biogenic polyamine complex, and further reduce impurities (proteins and inorganic salts, etc.). Furthermore, it is possible to further process chondroitin sulfate separately, for example, further purification, removal of chondroitin sulfate in the salt form, or depolymerization of chondroitin sulfate. In other embodiments, chondroitin sulfate and polyamine may be separated and extracted simultaneously.

In some specific embodiments, in the case of simultaneous separation and extraction of chondroitin sulfate and polyamine, the raw material is typically treated by enzymolysis. In some embodiments, an acid may be added to the enzymatic hydrolysate to precipitate the proteins in the enzymatic hydrolysate, so as to remove the proteins from the enzymatic hydrolysate. In some specific embodiments, after removal of the proteins from the enzymatic hydrolysate, the concentration of the chondroitin sulfate in the preliminary extraction solution may be in the range of about 6% to 7% (w/v). In this concentration range, it is more conducive to obtaining the chondroitin sulfate biogenic polyamine complex in the subsequent steps. The preliminary extraction solution containing chondroitin sulfate in the above concentration range contains a higher content of biogenic polyamine, which is more favorable to its complexation with chondroitin sulfate.

Furthermore, the liquid obtained after protein removal is concentrated and/or the pH value of the liquid is adjusted, and then ethanol is added for complex precipitation to obtain the chondroitin sulfate biogenic polyamine complex. In some specific embodiments, the acid for precipitating the proteins may be selected from trichloroacetic acid, perchloric acid, nitric acid, and the like.

In the case of stepwise separation and extraction of chondroitin sulfate and polyamine, in some embodiments, polyamine in the sample may be separated by chromatography or extraction, and then the residue is subjected to one or more of enzymolysis, protein precipitation method, chromatography, and alcohol precipitation method, so as to purify the chondroitin sulfate in the residue. The purified chondroitin sulfate is mixed with the separated polyamine, and added with ethanol for complex precipitation to obtain the chondroitin sulfate biogenic polyamine complex.

In some specific embodiments, the chromatography is ion-exchange chromatography. In some specific embodiments, the extraction is organic solvent extraction or supercritical extraction. In some more specific embodiments, the organic solvent used for the organic solvent extraction includes one or more of n-butanol, dichloromethane, chloroform, and ether. In other more specific embodiments, the supercritical extraction is supercritical carbon dioxide extraction.

In some specific embodiments, the stepwise separation and extraction of chondroitin sulfate and polyamine further comprises the step of depolymerizing the chondroitin sulfate. In some embodiments, for example, the chondroitin sulfate purified by one or more of enzymolysis, protein precipitation method, chromatography, and alcohol precipitation method is depolymerized by the method disclosed in CN111495428A (*e.g*., Example 2 of CN111495428A) to obtain low-molecular-weight chondroitin sulfate. In some embodiments, the low-molecular-weight chondroitin sulfate is mixed with the separated polyamine, and added with ethanol for complex precipitation to obtain the chondroitin sulfate biogenic polyamine complex.

In some specific embodiments, in the extract containing a chondroitin sulfate biogenic polyamine complex obtained by the above-mentioned method for preparing a chondroitin sulfate biogenic polyamine complex, the total polyamine content (mass ratio) is 0.01% to 5% as detected after separation of chondroitin sulfate.

### (Other Steps)

The other steps in the method for preparing a chondroitin sulfate biogenic polyamine complex are not particularly limited, and may be adjusted and selected depending upon the actual equipment or requirements. In some embodiments, the preparation method comprises the step of purifying the product to obtain a chondroitin sulfate biogenic polyamine complex with a higher purity. It can be appreciated, however, that the chondroitin sulfate biogenic polyamine complex prepared by the above method is already able to exert its functions.

In other embodiments of the present disclosure, the chondroitin sulfate biogenic polyamine complex provided herein can be prepared by mixing chondroitin sulfate and biogenic polyamine, wherein based on the total weight of the chondroitin sulfate biogenic polyamine complex, the chondroitin sulfate biogenic polyamine complex contains 0.5 to 200 µmol/g of the biogenic polyamine.

In some specific embodiments, the chondroitin sulfate and the biogenic polyamine are commercially available. In other specific embodiments of the present disclosure, the chondroitin sulfate and the biogenic polyamine can be extracted from the raw materials as described above.

In some more specific embodiments, the chondroitin sulfate and biogenic polyamine are prepared by mixing them in a solvent and then drying.

In some exemplary embodiments, the solvent is water. In some exemplary embodiments, the drying is freeze drying.

In some aspects of the present disclosure, there is further provided a chondroitin sulfate biogenic polyamine complex obtained by the above-mentioned preparation method.

### <Pharmaceutical Composition, Health Product and Cosmetic>

In some aspects of the present disclosure, there is further provided a pharmaceutical composition, comprising the above-mentioned chondroitin sulfate biogenic polyamine complex or a chondroitin sulfate biogenic polyamine complex obtained by the above-mentioned preparation method of a chondroitin sulfate biogenic polyamine complex, and a pharmaceutically acceptable carrier.

In other aspects of the present disclosure, there is further provided a health product, comprising the above-mentioned chondroitin sulfate biogenic polyamine complex or a chondroitin sulfate biogenic polyamine complex obtained by the above-mentioned preparation method of a chondroitin sulfate biogenic polyamine complex.

In other aspects of the present disclosure, there is further provided a cosmetic, comprising the above-mentioned chondroitin sulfate biogenic polyamine complex or a chondroitin sulfate biogenic polyamine complex obtained by the above-mentioned preparation method of a chondroitin sulfate biogenic polyamine complex.

### <Anti-Inflammatory Use and Method of Chondroitin Sulfate Biogenic Polyamine Complex>

In some aspects of the present disclosure, there is provided use of a chondroitin sulfate biogenic polyamine complex in the preparation of a drug for suppressing/anti-inflammation, wherein the chondroitin sulfate biogenic polyamine complex is the above-mentioned chondroitin sulfate biogenic polyamine complex or a chondroitin sulfate biogenic polyamine complex obtained by the above-mentioned preparation method of a chondroitin sulfate biogenic polyamine complex.

In some embodiments, there is provided use of a chondroitin sulfate biogenic polyamine complex in the preparation of a drug for treating and/or preventing an inflammatory disease, wherein the chondroitin sulfate biogenic polyamine complex is the above-mentioned chondroitin sulfate biogenic polyamine complex or a chondroitin sulfate biogenic polyamine complex obtained by the above-mentioned preparation method of a chondroitin sulfate biogenic polyamine complex.

In some embodiments of the present disclosure, the inflammatory disease is an inflammatory disease caused by various inflammation-inducing factors and/or mediated by inflammatory factors such as inflammatory cells, Interleukin (IL) and/or Tumor Necrosis Factor (TNF). In some specific embodiments, the inflammatory factors include either or both of Interleukin 6 (IL-6) and Interleukin 1β (IL-1β).

In some embodiments of the present disclosure, the inflammatory disease is a chronic inflammatory disease.

In some embodiments of the present disclosure, the inflammatory disease is one or more selected from allergy, eczema, myocardial infarction, cerebral infarction, Alzheimer's disease, dermatitis or arthritis.

In some embodiments of the present disclosure, the inflammatory disease is arthritis. In some more specific embodiments of the present disclosure, arthritis is chronic arthritis such as multiple arthritis. More specifically, arthritis is chronic rheumatoid arthritis.

In some aspects of the present disclosure, there is provided a method of treating and/or preventing an inflammatory disease, comprising a step of administering, to a subject, a therapeutically effective amount or a prophylactically effective amount of a chondroitin sulfate biogenic polyamine complex, wherein the chondroitin sulfate biogenic polyamine complex is the above-mentioned chondroitin sulfate biogenic polyamine complex or a chondroitin sulfate biogenic polyamine complex obtained by the above-mentioned preparation method of a chondroitin sulfate biogenic polyamine complex.

### <Blood Lipids-Lowing Use and Method of Chondroitin Sulfate Biogenic Polyamine Complex>

In other aspects of the present disclosure, it has also been found that the above-mentioned chondroitin sulfate biogenic polyamine complex or a chondroitin sulfate biogenic polyamine complex obtained by the above-mentioned preparation method of a chondroitin sulfate biogenic polyamine complex has the effect of lowering blood lipids.

Therefore, in some embodiments, there is provided use of a chondroitin sulfate biogenic polyamine complex in the preparation of a drug for lowering blood lipids; in the other embodiments, there is provided use of a chondroitin sulfate biogenic polyamine complex in the preparation of a drug for treating and/or preventing hyperlipidemia; wherein the chondroitin sulfate biogenic polyamine complex is the above-mentioned chondroitin sulfate biogenic polyamine complex or a chondroitin sulfate biogenic polyamine complex obtained by the above-mentioned preparation method of a chondroitin sulfate biogenic polyamine complex.

In some specific embodiments, hyperlipidemia may be primary hyperlipidemia and/or secondary hyperlipidemia. In some specific embodiments, hyperlipidemia may be hypertriglyceridemia and/or hypercholesterolemia.

In some specific embodiments, the drug for treating and/or preventing hyperlipidemia is also applicable to the conditions associated with (*e.g*., triggered or exacerbated by) hyperlipidemia, including a cardiovascular disease, for example, but not limited to, arteriosclerosis, coronary artery disease, angina pectoris, carotid artery disease, stroke, cerebral arteriosclerosis, myocardial infarction, cerebral infarction, restenosis after balloon angioplasty, hypertension, intermittent claudication, dyslipidemia, postprandial lipemia, and xanthoma.

In some aspects of the present disclosure, there is provided a method of treating and/or preventing hyperlipidemia, comprising a step of administering, to a subject, a therapeutically effective amount or a prophylactically effective amount of a chondroitin sulfate biogenic polyamine complex, wherein the chondroitin sulfate biogenic polyamine complex is the above-mentioned chondroitin sulfate biogenic polyamine complex or a chondroitin sulfate biogenic polyamine complex obtained by the above-mentioned preparation method of a chondroitin sulfate biogenic polyamine complex.

In further aspects of the present disclosure, there is provided use of a chondroitin sulfate biogenic polyamine complex in the preparation of a health food for regulating blood lipids (lowering total cholesterol or lowering triglycerides) or assisting in lowering the blood lipids, wherein the health food helps maintain a healthy level of blood lipids (cholesterol/triglycerides); wherein the chondroitin sulfate biogenic polyamine complex is the above-mentioned chondroitin sulfate biogenic polyamine complex or a chondroitin sulfate biogenic polyamine complex obtained by the above-mentioned preparation method of a chondroitin sulfate biogenic polyamine complex.

### <Other Uses of Chondroitin Sulfate Biogenic polyamine Complex>

The present disclosure has also found that the above-mentioned chondroitin sulfate biogenic polyamine complex or a chondroitin sulfate biogenic polyamine complex obtained by the above-mentioned preparation method of a chondroitin sulfate biogenic polyamine complex has the effects of treating/repairing the joint injury, anti-oxidation, delaying aging, extending lifespan, etc.

Therefore, in some embodiments, there is provided use of a chondroitin sulfate biogenic polyamine complex in the preparation of a drug for treating and/or repairing a joint injury, wherein the chondroitin sulfate biogenic polyamine complex is the above-mentioned chondroitin sulfate biogenic polyamine complex or a chondroitin sulfate biogenic polyamine complex obtained by the above-mentioned preparation method of a chondroitin sulfate biogenic polyamine complex.

In some specific embodiments, the joint injury may be a joint injury caused by inflammation, aging, exercise, injury, etc.

In the other aspects of the present disclosure, there is provided use of a chondroitin sulfate biogenic polyamine complex in the preparation of a health food; wherein the chondroitin sulfate biogenic polyamine complex is the above-mentioned chondroitin sulfate biogenic polyamine complex or a chondroitin sulfate biogenic polyamine complex obtained by the above-mentioned preparation method of a chondroitin sulfate biogenic polyamine complex. In some specific embodiments, there is provided use of a chondroitin sulfate biogenic polyamine complex in the preparation of a health food that contributes to an improvement of the bone mineral density.

In some embodiments, there is provided use of a chondroitin sulfate biogenic polyamine complex in the preparation of a drug for anti-oxidation, delaying aging and/or extending lifespan, wherein the chondroitin sulfate biogenic polyamine complex is the above-mentioned chondroitin sulfate biogenic polyamine complex or a chondroitin sulfate biogenic polyamine complex obtained by the above-mentioned preparation method of a chondroitin sulfate biogenic polyamine complex.

In some specific embodiments, the drug achieves an antioxidant effect by scavenging oxygen free radicals.

In further aspects of the present disclosure, there is provided use of a chondroitin sulfate biogenic polyamine complex in the preparation of a health food that contributes to anti-oxidation, wherein the health food helps the human body maintain a balance between oxidative and antioxidant processes; wherein the chondroitin sulfate biogenic polyamine complex is the above-mentioned chondroitin sulfate biogenic polyamine complex or a chondroitin sulfate biogenic polyamine complex obtained by the above-mentioned preparation method of a chondroitin sulfate biogenic polyamine complex.

In further aspects of the present disclosure, there is provided use of a chondroitin sulfate biogenic polyamine complex in the preparation of a cosmetic, wherein the chondroitin sulfate biogenic polyamine complex is the above-mentioned chondroitin sulfate biogenic polyamine complex or a chondroitin sulfate biogenic polyamine complex obtained by the above-mentioned preparation method of a chondroitin sulfate biogenic polyamine complex. In some specific embodiments, there is provided use of a chondroitin sulfate biogenic polyamine complex in the preparation of a cosmetic that contributes to anti-oxidation.

### Examples and Test Examples

The present disclosure is further illustrated below with reference to examples and test examples which, however, are not intended to limit the present disclosure. Specific materials used in the embodiments of the present disclosure and their sources are provided below. It should be appreciated, however, that these materials are merely exemplary and are not intended to limit the present disclosure, and materials of the same or similar type, model, quality, properties or functions as the following reagents and instruments can all be used to implement the present disclosure. All the experimental methods used in the following examples and test examples are conventional methods, unless otherwise specified. Materials, reagents and the like used in the following examples and test examples are all commercially available, unless otherwise specified.

In the following examples, the molecular weight of the chondroitin sulfate/chondroitin sulfate biogenic polyamine complex is determined by the following method:
The molecular weight and the molecular weight distribution of the chondroitin sulfate/chondroitin sulfate biogenic polyamine complex are determined by high-performance Gel Permeation Chromatography (GPC). The dextran molecular weight standard serves as a standard. The mobile phase is 0.2 M aqueous sodium sulfate solution.

The chromatographic conditions are as follows:
Chromatographic column: TSKgel G3000PWXL 7.8*300 mm
Flow rate: 0.5 mL/min
Detector: differential refractive index detector
Column temperature: 35°C
Sample volume: 30 µL.

In the following examples, the polyamine assay is carried out in accordance with GB5009.208-2016.

The examples below adhere to the following fundamental principle: Because of a strong non-covalent bonding force between biogenic polyamine and chondroitin sulfate, the conditions such as pH and concentrations of biogenic polyamine and chondroitin sulfate in the solution are adjusted to make them precipitate by alcohol precipitation with ethanol, thereby fulfilling the extraction and purification purposes. However, due to the presence of a large number of other molecules (amino acids, peptides, proteins, fats, inorganic salts, etc.) in biological tissue, in order to eliminate the interference of other substances and further improve the purity and the polyamine content, different extraction processes are adopted depending on different raw materials and requirements.

### Examples: Extraction of Chondroitin Sulfate Biogenic Polyamine Complex

### Example 1. Discovery and Verification of Chondroitin Sulfate Biogenic Polyamine Complex

In this example, the chondroitin sulfate biogenic polyamine complex, as a whole, was separated and precipitated from animal bone tissue. This example conducted analyses and characterizations from many aspects before confirming that the source of activity was the chondroitin sulfate polyamine substance.

### I. Extraction

In this example, chicken bones were used as raw material.

### (1) Pretreatment of raw material

Fresh raw bones were boiled to remove impurities such as blood, grease, and residual meat. Afterwards, the bones were washed with clean water, and then dried, ground and pulverized.

### (2) Separation and extraction of chondroitin sulfate and polyamine in raw material

### 2.1 Enzymolysis

The pretreated chicken bones were weighed, 5 g for each. 100 mL of water at pH 5.7-6.0 and 2% of enzyme based on the mass of the raw material chicken bones were added, respectively. The enzymes were as follows:
0.1 g of papain (200 U/mg, Aibisheng Biotechnology) **(A-2);**
0.1 g of papain (2000 U/mg, Adamas reagent) **(A-3);**
0.1 g of papain (800 U/mg, Aibisheng Biotechnology) **(A-4);** or
0.1 g of trypsin (250 U/mg, Aladdin reagent) and 0.1 g of pepsin (3000 U/mg, Aladdin reagent) **(A-6).**

After mixed well with stirring, the system was heated to 65°C, and reacted for 3 h at the same temperature. The pretreated chicken bones were subjected to enzymolysis to form an enzymatic hydrolysate.

### 2.2 Separation and extraction of chondroitin sulfate and polyamine

Upon completion of the enzymolysis reaction, the enzymatic hydrolysates obtained in step 2.1 were filtered through filter paper, respectively. 5.0 g of trichloroacetic acid (Aladdin reagent, China) was added to the filtrates obtained after filtration respectively, placed in ice bath for 1 h, mixed evenly, and centrifuged at 8000 rpm/min for 5 min. The supernatant was collected to remove excess proteins.

### (3) Complex precipitation into chondroitin sulfate biogenic polyamine complex

The supernatant obtained in step 2.2 was adjusted to about pH5 with a sodium hydroxide solid respectively, and concentrated under reduced pressure to approximate 10 mL of yellow liquid (without precipitation of solids) respectively.

The yellow liquid was added with 25 mL of anhydrous ethanol respectively, subjected to alcohol precipitation, oscillation, and centrifugation to obtain an extract (the chondroitin sulfate biogenic polyamine complex) respectively. The alcohol precipitated solid obtained after centrifugation was washed once with anhydrous ethanol again, and centrifuged. The solid obtained after centrifugation was vacuum dried overnight at 40°C to yield 600 mg of white solid as the extract (which was subsequently verified as the chondroitin sulfate biogenic polyamine complex).

### II. Assay of Extract

The above extracts obtained by extraction after treatment with different enzymes were assayed and verified.

To begin with, the inhibitory level (*i.e.,* the anti-inflammatory activity) of the extract against IL-6 was tested. Specifically, the cells used were mouse monocytes RAW264.7. The cells were digested and inoculated into a 96-well plate, with 10,000 cells per well. After cell adhesion, Dexamethasone (DXM) and CSX were diluted with a DMEM 10% FBS medium containing 0.1 µg/ml LPS. The final Dexamethasone concentration was 0.1 mg/ml, and the final CSX concentration was 0.1 mg/ml. The cells were cultured for 18 to 24 h. The supernatant was aspirated. The level of IL-6 in the supernatant was tested by the enzyme linked immunosorbent assay (Elisa).

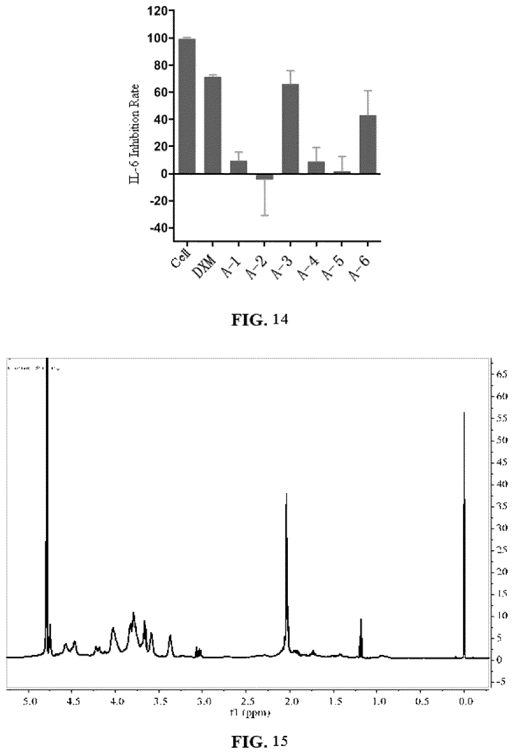

FIG. 14 was a diagram showing the inhibitory levels of IL-6 after the action of different extracts and existing chondroitin, in which A-2, A-3, A-4, and A-6 represented the extracts obtained by extraction after treatment with the above different enzymes. A-1 represented chondroitin provided by a corporation (Wuxing Biotechnology, Hunan Province, China), and A-5 represented chondroitin purchased from a reagent company (Aladdin, Shanghai, China). As could be appreciated from FIG. 14, the inhibitory effect of Extract A-3 on IL-6 was significantly better than those of the other extracts or chondroitin, followed by Extract A-6, while there was no significant difference among Chondroitin A-1, A-4, and A-5.

The chondroitin extract (A-3) with a better anti-inflammatory activity was selected for the H NMR spectrum test (solvent: deuterated water, 600 MHZ, Agilent, U.S.A.). It was found that in addition to the hydrogen atom signals of the chondroitin molecules, obvious new signals occurred at chemical shifts 1.7, 1.9 and 3.0 ppm (FIG. 15).

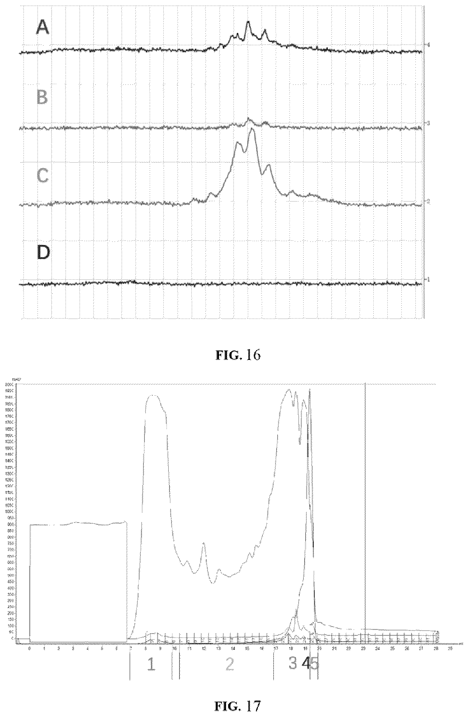

FIG. 16 was a localized close-up of the characteristic peak to (1.7 ppm) in the H NMR spectra of different extracts. In FIG. 16, A, B, C, and D were extracts obtained by the above different extraction methods, and corresponded to Extract A-6, Extract A-4, Extract A-3, and Extract A-2, respectively. The relative integral areas were C > A > B > D, and the order of the inhibitory activities of the corresponding extracts against the inflammatory factor was A-3 > A-6 > A-4 > A-2. The integral area of the characteristic peak corresponded to the magnitude of the activity.

In order to further explore the source of activity, attempts were made to separate the components in the extract and separate chondroitin from the substance at chemical shifts 1.7, 1.9 and 3.0 ppm. The separation method was as follows:
To 2 L of pure water, 100 g of Extract A-3 and 200 g of Hexadecylpyridinium Chloride (CPC) (InnoChem Corporation, LOT: KYGA540) were added. Chondroitin and CPC underwent an electrostatic interaction to produce a white precipitate. After stirring at room temperature for 1 h, the system was centrifuged (7000 rpm, 10°C, 15 min). The precipitate was a complex of CPC and chondroitin, while the clear solution contained the components of the substance at chemical shifts 1.7, 1.9, and 3.0 ppm. The chondroitin and CPC precipitated, added to 10% aqueous NaCl solution, and heated and stirred at 60°C to dissociate the precipitate. Thereafter, three times the solution volume of anhydrous ethanol was added, and the precipitate was chondroitin sulfate. After the ethanol precipitation operation was repeated twice, the precipitate was filtered and then dried to yield 81 g of a white solid with a purity of 95.5%. NMR test revealed that the characteristic peaks of the above chemical shifts disappeared.

The above-mentioned clear solution containing the components of the substance at chemical shifts 1.7, 1.9, and 3.0 ppm was washed three times with dichloromethane (200 mL/time) to remove excess CPC. The aqueous phases were collected. Approximate 280 mL of anhydrous ethanol was added. A precipitate (substances such as salts, proteins, and excess chondroitin) appeared, and removed by centrifugation. The clear solution was freeze dried for 48 h to obtain a white solid. The white solid was dissolved, and then further separated using a gel chromatography column under the following separation conditions: The separation equipment was AKTA pure^{™} protein purification system (U.S.A.), and the chromatographic column was GE Superdex G30 increase 10/300 GL (U.S.A.). The mobile phase was water with a flow rate of 0.5 ml/min. The detection wavelengths were 210 nm and 260 nm. The fractions were collected separately (the chromatogram was shown in FIG. 17, in which the digits were the serial numbers of the collected fractions), and again subjected to the test on the activity against the anti-inflammatory factor IL-6 respectively by the above-mentioned method. The activity of No. 3 fraction was found to be much higher than those of the other components. After Fraction A-3 was further concentrated and determined by nuclear magnetic resonance and mass spectrometry, it was confirmed as three biogenic polyamines: spermine, spermidine, and putrescine, and the molar ratio of these three amines was close to 1:1:1.

In order to further demonstrate the form in which CS and polyamines were present in the extract, the following experiment was carried out and confirmed that CS and polyamine were bound to each other:
1. No. 3 extract was dialyzed for 5 days in a dialysis bag with a molecular weight cut-off of 10 kD, and water was replaced every 8 h. At the end of dialysis, the retentate was freeze dried. After testing the polyamine content, it was found that the polyamine content did not reduce.
2. Reaction of fluorescein isothiocyanate (FITC) and chondroitin polyamine

Experimental method: Extract A-3 (50 mg) was dissolved in 10 mL of pure water, and adjusted with 6 mol/L sodium hydroxide solution to pH 9-10. Thereafter, an aqueous solution containing 10 mg of FITC (1 mL) was added. Wrapped in a tin foil, the system was reacted overnight at room temperature in a dark place. After dialysis for 5 days with a 1000D dialysis membrane, three times the volume of anhydrous ethanol was added to yield a yellow precipitate. After filtration, the yellow precipitate was washed once with anhydrous ethanol to obtain a yellow solid, and the yellow solid was vacuum dried overnight. The same operations were conducted using ordinary chondroitin as a control. Ordinary chondroitin was white, while the active chondroitin was yellow, so a significant difference in color could be observed. This indicated that a condensation reaction occurred between the FITC molecules and the amino groups of the polyamine molecules. Although the binding ability of some of the amino groups to CS was weakened as a result of forming amides, there were still unreacted amino groups that had strong binding action with CS. Since the polyamine molecules remained bound to the polymer chains of CS, there was still visible color after dialysis. However, ordinary chondroitin was unable to react with FITC molecules, so the free FITC molecules were removed. Ordinary chondroitin was the bovine-derived chondroitin provided by Hunan Wuxing Biological Technology Co., Ltd., and had a weight-average molecular weight of 21k, among which the proportion of chondroitin sulfate having a molecular weight of greater than 50000 was 0%, the proportion at 25000 to 50000 was 31%, the proportion at 400 to 25000 was 69%, and the proportion of chondroitin sulfate having a molecular weight of 400 or less was 0%.

### 3. ITC reaction

To detect whether there was affinity between the chondroitin molecules and the polyamine molecules and the magnitude of affinity, Isothermal Titration Calorimetry (ITC) (MicroCal^{™} iTC200) was adopted to determine the binding constant of the chondroitin molecules to the polyamine molecules.

Polyamine molecules were used a ligand for titration of chondroitin molecules and the parameters were set according to a binding molar ratio of 1:1. The chondroitin molecules were dissolved in water at a concentration of 20 µM, and the polyamine molecules were also dissolved in water at a concentration of 200 µM. A total of 17 times of titration was conducted at 2 µL per drop.

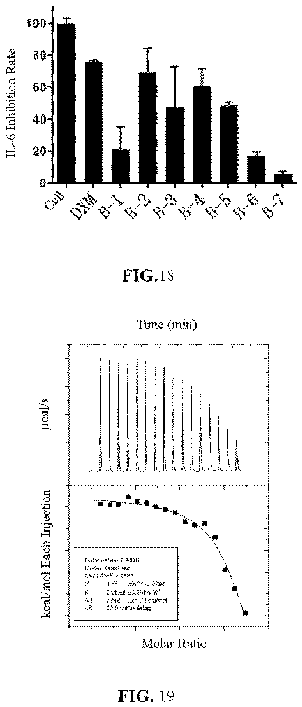

The experimental results were as shown in FIG. 19. The binding constant of the chondroitin molecules to the polyamine molecules was KD = 1/KA = 5 µM, the reaction stoichiometry was N = 1.74, and the enthalpy was ΔH = 2.29 Kcal/mol. Hence, there was a strong binding force between the chondroitin molecules and the polyamine molecules.

The above test showed that the main active ingredient in Extracts A-2, A-3, A-4, and A-6 was the chondroitin sulfate biogenic polyamine complex containing spermine, spermidine, and putrescine. After separation and detection, based on the total weight of the extract, the results of the polyamine content (molar mass ratio), the mass ratio of chondroitin sulfate, the mass ratio of protein, and the weight-average molecular weight were listed in Table 1 below. The mass ratio of chondroitin sulfate was tested with reference to the method of sodium chondroitin sulfate in the Chinese Pharmacopoeia 2020*,* and the standard sample was chondroitin sulfate (National Institutes for Food and Drug Control). The protein content was assayed by Lowry, and the molecular weight was determined by GPC. The reason for larger molecular weights of A-2 and A-4 might be incomplete destruction of the covalent bonds that link the protein to multiple polysaccharide molecules. The presence of protein may lead to a reduced anti-inflammatory activity of the complex, or even a pro-inflammatory effect. For example, A-2 exhibited a certain pro-inflammatory effect, which might be caused by the excessively high content of impurities such as proteins. However, A-6 did not exhibit a good anti-inflammatory effect, which might also be caused by a higher protein content.

**Table 1:**

| | Polyamine (Molar Mass Ratio: µmol/g) | | | | Weight-Average Molecular Weight | Protein Content (%) | Chondroitin content (%) |
|---|---|---|---|---|---|---|---|
| | Putrescine | Spermine | Spermidine | Total content | | | |
| A-2 | 0.062 | 0.086 | 0.039 | 0.188 | 54k | 38.9 | 50.1 |
| A-3 | 0.602 | 0.574 | 0.589 | 1.785 | 25k | 3.1 | 84.1 |
| A-4 | 0.060 | 0.144 | 0.118 | 0.323 | 35k | 19.4 | 75.8 |
| A-6 | 0.124 | 0.316 | 0.275 | 0.715 | 28k | 10.2 | 70.7 |

In order to further validate the source of activity, compounding was carried out according to the ratio of polyamines in Extract A-3. Each of the three polyamines was also compounded with chondroitin in an equimolar weight, and their inhibitory abilities against IL-6 were determined altogether by the method as described previously. The compounding method was as follows: after chondroitin sulfate (Sigma, U.S.A.) was dissolved into water, spermine tetrahydrochloride (Acros, U.S.A.), spermidine trihydrochloride (Acros, U.S.A.), and putrescine dihydrochloride (Acros, U.S.A.) were added separately or simultaneously, stirred thoroughly, and then freeze dried to obtain a white solid. The weight-average molecular weight of chondroitin sulfate here was 20100. The proportion of chondroitin sulfate having a molecular weight of 400 to 25000 was 71%, the proportion at 25000 to 50000 was 29%, and the proportion at 400 or less was 0%.

FIG. 18 showed the test on the anti-inflammatory activities of various substances against IL-6. B-1 was a combination of biogenic polyamines (spermine + spermidine + putrescine; the ratio of the three polyamines was the same as that in Extract B-4; the total molar weight of the three polyamines was the same as that of the polyamines contained in the amount of Extract B-4). B-2 was a complex of CS with a combination of polyamines (CS + spermine + spermidine + putrescine; the ratio of the three polyamines was the same as that in Extract B-4; the total molar weight of the three polyamines was the same as that of the polyamines contained in Extract B-4; the amount of CS was the same as the mass of Extract B-4 minus polyamines). B-3 was CS + spermine (the molar weight of spermine was the same as the total molar weight of polyamines contained in Extract B-4; the amount of CS was the same as the mass of Extract B-4 minus polyamines). B-4 was Extract A-3 described above. B-5 was CS + spermidine (the molar weight of spermidine was the same as the total molar weight of polyamines contained in Extract B-4; the amount of CS was the same as the mass of Extract B-4 minus polyamines). B-6 was CS + putrescine (the molar weight of putrescine was the same as the total molar weight of polyamines contained in the amount of Extract B-4; the amount of CS was the same as the mass of Extract B-4 minus polyamines). B-7 was CS (its amount was the same as the mass of Extract B-4). The above results showed that in the case of the same content of polyamines, the activity of the compound was comparable to that of the extract, higher than that of the above biogenic polyamine composition (not compounded with CS) at the equal amount, and much higher than that of a single CS. The proportion of the total molar weight of polyamines in the compound was the same as that in A-3.

The above results showed that in the case of the same content of polyamines, the activity of the compound was comparable to that of the extract, and its inhibition rate against the inflammatory factor was about 3.5 times higher than that of the above biogenic polyamine composition (not compounded with CS) at the equal amount, and much higher than that of a single CS.

In the existing extraction methods for chondroitin sulfate, the other components were usually removed from the raw material in order to obtain chondroitin sulfate with a higher purity. Unlike the prior art, the present disclosure had unexpectedly obtained a complex of chondroitin sulfate and polyamines by precipitating chondroitin sulfate with anhydrous ethanol under certain conditions after adequate action of enzymes on the raw material, and found that the complex has an unexpectedly significantly improved activity compared to chondroitin sulfate. Therefore, in the subsequent examples, a stepwise extraction method was adopted to increase the content of polyamines obtained from the raw material, and complex them with the chondroitin sulfate obtained from the raw material, so as to obtain the chondroitin sulfate polyamine from natural raw materials.

### Example 2. Papain Enzymolysis-Purification and Extraction of Resin + Complex Precipitation with Anhydrous Ethanol

In this example, porcine cartilage was used as the raw material for preparing the chondroitin sulfate biogenic polyamine complex.

### (1) Pretreatment of raw material

The raw material was pretreated by the same method described in Example 1.

### (2) Separation and extraction of chondroitin sulfate and polyamine in raw material

### 2.1 Enzymolysis

100 g of pretreated porcine cartilage was weighed, and 500 mL of water and 2% of papain (2000 U/mg) based on the mass of the raw material porcine cartilage. The system was adjusted to pH 6-7, heated to 55°C, and stirred for 16 h. The enzymolysis system was cooled to room temperature to obtain an enzymolysis product.

### 2.2 Chromatography of biogenic polyamine

The enzymolysis product obtained in 2.1 was subjected to vacuum suction filtration on diatomite to remove insoluble impurities such as bone residues to yield a yellowish transparent solution.

100 mL of weak acidic cation exchange resin (Type D152 in this example) was measured and added into an empty column tube, which was rinsed with pure water and filled. 500 mL of the above yellowish transparent solution (filtrate) passed through the column, and the permeate was collected. Thereafter, the resin column was rinsed with 200 mL of 4% (m/v) dilute hydrochloric acid, and the eluent was collected as eluent A. After adsorption and elution through the cation exchange column as described above, the polyamine substance could be concentrated and purified, and enriched in eluent A.

### 2.3 Separation and extraction of chondroitin sulfate

The permeate obtained in step 2.2 was distillated under reduced pressure, and concentrated to 100 mL. 5 g of trichloroacetic acid was added to precipitate proteins. The precipitate was removed by centrifugation, and the clear solution was retained.

The permeate treated with protein precipitation method by trichloroacetic acid was added with three times the volume of anhydrous ethanol for alcohol precipitation to obtain chondroitin sulfate, oscillated, and centrifuged. The lower-layer solid obtained by centrifugation was washed once with anhydrous ethanol, and centrifuged. The solid obtained after centrifugation was vacuum dried overnight at 45°C to obtain a crude sodium chondroitin sulfate product.

In order to further purify chondroitin sulfate, the resulting crude sodium chondroitin sulfate product was formulated into an aqueous solution at a mass/volume percent of 20%, added with 50 mL of strong basic anion exchange resin (D280 in this example), and adsorbed with stirring for 20 to 30 min. Afterwards, 1 to 1.5 times the resin volume of 9% (m/v) sodium chloride solution was added for elution to obtain eluent B. The eluent B was subjected to alcohol precipitation again with three times the volume of anhydrous ethanol to precipitate chondroitin sulfate, oscillated, and centrifuged. The lower-layer solid obtained by centrifugation was washed once with anhydrous ethanol, and centrifuged. The solid obtained after centrifugation was vacuum dried overnight at 45°C to obtain sodium chondroitin sulfate. The purity was determined to be 90.1% by the same method described in Example 1.

### (3) Depolymerization of chondroitin sulfate (catalytic degradation of solid phase)

The dried solid (sodium chondroitin sulfate) obtained in step 2.3 was dissolved in 100 mL of water to obtain a solution, and the solution was treated according to CN111495428A (Title of Patent: Method for Preparing Low-Molecular-Weight Polysaccharide and Catalyst Used in Method): in brief, 0.1 g of resin catalyst and 2.1 mL of 30% hydrogen peroxide were added to the solution, and reacted at 50°C for 6 h to obtain a low-molecular weight chondroitin sulfate solution.

### (4) Complex precipitation into chondroitin sulfate biogenic polyamine complex

To the low-molecular-weight chondroitin sulfate solution obtained in step (3), the eluent A obtained in step 2.2 was added, and the pH was adjusted to about 5. Then, 2.5 times the volume of anhydrous ethanol was added for alcohol precipitation, oscillated, and centrifuged. The lower-layer solid obtained by centrifugation was washed once with anhydrous ethanol, and centrifuged. The solid obtained after centrifugation was vacuum dried overnight at 45°C to obtain 6.0 g of milky white solid as the chondroitin sulfate biogenic polyamine complex.

The above substance was tested as a chondroitin sulfate biogenic polyamine complex containing spermine and spermidine. After separation and detection, the total polyamine content (molar mass ratio) in the extract was 23.26 µmol/g, including 4.31 µmol/g of spermine, 7.07 µmol/g of spermidine, 5.59 µmol/g of putrescine, and 6.29 µmol/g of cadaverin. The low-molecular chondroitin had a weight-average molecular weight of 4.3k, among which the proportion at greater than 50000 was 0%, the proportion at 25000 to 50000 was 0%, and the proportion at 400 to 25000 was 100%. Specifically, the content of chondroitin having a molecular weight of 8000 to 25000 was 14%, the content at 5000 to 8000 was 30%, the content at 400 to 5000 was 56%, and the content at 400 or less was 0%.

### Example 3. Acid Extraction-Organic Solvent Extraction + Complex Precipitation with Anhydrous Ethanol

In this example, chicken tissue (containing chicken bones, heads, and viscera) was used as the raw material for preparing the chondroitin sulfate biogenic polyamine complex.

### (1) Pretreatment of raw material

Meat was removed from fresh chicken tissue (containing chicken bones, heads, and viscera).

### (2) Separation and extraction of chondroitin sulfate and polyamine in raw material

### 2.1 Acidolysis

100 g of pretreated chicken tissue (containing chicken bones, heads, and viscera) was weighed, ultrasonically extracted twice with 1 M dilute hydrochloric acid for a total of 2 h (300 mL 1 h, 200 mL 1 h), and then filtered through filter paper. The filter tissue residues were washed twice with 50 mL of pure water, and the tissue residues were set aside.

### 2.2 Extraction of biogenic polyamine

All the liquids obtained in step 2.1 were combined to obtain a total of 600 mL of slightly turbid yellowish solution. The solution was concentrated to 50 mL by rotary evaporation, and centrifuged. The supernatant was collected, adjusted with sodium hydroxide to about pH12, and centrifuged. The supernatant was extracted twice with 10 mL of n-butanol to extract a mixture of biogenic polyamines. The organic phases were combined, adjusted with dilute hydrochloric acid to acidity, concentrated to dryness to obtain a gray-brown solid as a crude extract of biogenic polyamines.

### 2.3 Separation and extraction of chondroitin sulfate

### 2.3.1 Enzymolysis

To the tissue residues obtained in step 2.1, 100 mL of water at pH 5.7-6.0 and 2% of papain (2000 U/mg) based on the mass of the tissue residues were added, and stirred evenly. The system was heated to 65°C and reacted for 3 h at the same temperature. Thereafter, 1 g of papain was supplemented and further reacted for 3 h to obtain an enzymatic hydrolysate.

### 2.3.2 Separation and extraction

After the enzymatic hydrolysate obtained in step 2.3.1 was filtered, 20.0 g of trichloroacetic acid was added to the filtered filtrate, temporarily stored in ice bath for 1 h, mixed evenly, and centrifuged at 8000 rpm/min for 5 min. The supernatant was collected to remove proteins. The supernatant was adjusted with a sodium hydroxide solid to about pH5, and three times the volume of anhydrous ethanol was added for alcohol precipitation, oscillated, and centrifuged. The filtrate was stored temporarily. The alcohol precipitated solid obtained by centrifugation was washed once with anhydrous ethanol again, and centrifuged. The solid obtained by centrifugation was vacuum dried overnight at 40°C to obtain a white solid as chondroitin sulfate.

### (3) Depolymerization of chondroitin sulfate (catalytic degradation of solid phase)

The dried white solid (chondroitin sulfate) obtained in step 2.3.2 was dissolved in 100 mL of water to obtain a solution, and the solution was depolymerized according to CN111495428A (Method for Preparing Low-Molecular-Weight Polysaccharide and Catalyst Used in Method) to obtain a low-molecular-weight chondroitin sulfate solution. In brief, 0.05 g of resin catalyst and 1.1 mL of 30% hydrogen peroxide were added to the solution, and reacted at 50°C for 3 h to obtain a low-molecular-weight chondroitin sulfate solution.

### (4) Complex precipitation into chondroitin sulfate biogenic polyamine complex

The low-molecular-weight chondroitin sulfate solution obtained in step (3) was used to dissolve the crude extract of biogenic polyamine (a gray-brown solid) obtained in step 2.2, and the resulting solution was adjusted to about pH5. 2.5 times the volume of anhydrous ethanol was added for alcohol precipitation, oscillated, and centrifuged. The lower-layer solid obtained by centrifugation was washed once with anhydrous ethanol, and centrifuged. The solid obtained by centrifugation was vacuum dried overnight at 45°C to obtain 6.0 g of milky white solid as the chondroitin sulfate biogenic polyamine complex.

The above substance was tested as a chondroitin sulfate biogenic polyamine complex containing spermine, spermidine, putrescine, and cadaverin. After separation and detection, the total polyamine content (molar mass ratio) in the extract was 56.91 µmol/g, including 28.16 µmol/g of spermine, 9.82 µmol/g of spermidine, 8.07 µmol/g of putrescine, and 10.86 µmol/g of cadaverin. The low-molecular chondroitin had a weight-average molecular weight of 6.2k, among which the proportion at greater than 50000 was 0%, the proportion at 25000 to 50000 was 0%, and the proportion at 400 to 25000 was 100%. Specifically, the proportion at 8000 to 25000 was 24%, the proportion at 5000 to 8000 was 26%, the proportion at 400 to 5000 was 43%, and the proportion at 400 or less was 0%.

### Example 4. Acid Extraction-Organic Solvent Extraction + Complex Precipitation with Anhydrous Ethanol

In this example, fishbone and soybeans were used as raw materials for preparing a chondroitin sulfate biogenic polyamine complex. Soybeans were rich in spermine and spermidine. The purpose of adding soybeans as the raw material was to increase the polyamine content in the product.

### (1) Pretreatment of raw material

The fishbone and soybeans as raw materials were pulverized by a blender.

### (2) Separation and extraction of chondroitin sulfate and polyamine in raw material

### 2.1 Acidolysis

100 g of pretreated fishbone and 10 g of pretreated soybeans were weighed, ultrasonically extracted twice with 1 M dilute hydrochloric acid for a total of 2 h (300 mL 1 h, 200 mL 1 h), and then filtered through filter paper. The filter tissue residues were washed twice with 50 mL of pure water, and the tissue residues were set aside.

### 2.2 Extraction of biogenic polyamine

All the liquids obtained in step 2.1 were combined to obtain a total of 600 mL of slightly turbid yellowish solution, concentrated to 50 mL by rotary evaporation, and centrifuged. The supernatant was collected, adjusted with sodium hydroxide to about pH12, and centrifuged. The supernatant was extracted twice with 10 mL of n-butanol to extract a mixture of biogenic polyamines. The organic phases were combined, and concentrated to dryness to obtain a gray-brown solid as a crude extract of biogenic polyamine.

### 2.3 Separation and extraction of chondroitin sulfate

### 2.3.1 Enzymolysis

To the tissue residues obtained in step 2.1, 100 mL of water at pH 5.7-6.0 and 2% of papain (2000 U/mg) based on the mass of the tissue residues were added, and stirred evenly. The system was heated to 65°C and reacted for 3 h at the same temperature. Thereafter, 1 g of papain was supplemented and further reacted for 3 h to obtain an enzymatic hydrolysate.

### 2.3.2 Separation and extraction

The enzymatic hydrolysate obtained in step 2.3.1 was filtered through filter paper. To the filtered filtrate, 20.0 g of trichloroacetic acid was added, temporarily stored in ice bath for 1 h, mixed evenly, and centrifuged at 8000 rpm/min for 5 min. The supernatant was collected to remove proteins. 100 mL of strong acidic cation exchange resin (Type 001*7 in this example) was measured and added into an empty column tube, which was rinsed with pure water and filled. The centrifuged supernatant passed through the column, and the permeate was collected. The permeate was chondroitin sulfate acid.

### (3) Complex precipitation into chondroitin sulfate biogenic polyamine complex

The gray-brown solid obtained in step 2.2 was dissolved in the permeate obtained in step 2.3.2, and the resulting solution was adjusted to pH5.0. 2.5 times the volume of anhydrous ethanol was added for alcohol precipitation, oscillated, and centrifuged. The alcohol precipitated solid obtained by centrifugation was washed once with anhydrous ethanol again, and centrifuged. The solid obtained by centrifugation was vacuum dried overnight at 40°C to obtain a white solid as the chondroitin sulfate biogenic polyamine complex.

The above substance was tested as a chondroitin sulfate biogenic polyamine complex containing spermine, spermidine, putrescine, and cadaverin. After separation and detection, the total polyamine content (molar mass ratio) in the extract was 129.7 µmol/g, including 5.2 µmol/g of spermine, 70.3 µmol/g of spermidine, 49.7 µmol/g of putrescine, and 4.6 µmol/g of cadaverin. The chondroitin had a weight-average molecular weight of 22k, among which the proportion at greater than 50000 was 0%, the proportion at 25000 to 50000 was 37%, and the proportion at 400 to 25000 was 63%. Among them, the proportion at 8000 to 25000 was 53%, the proportion at 5000 to 8000 was 8%, the proportion at 400 to 5000 was 2%, and the proportion at 400 or less was 0%.

### Test Examples

### Test Example 1: Evaluation of Therapeutic Effects of Different Chondroitin Sulphates on Chronic Inflammation by Adjuvant Arthritis Mouse Test

To analyze whether CSX could inhibit the inflammatory response in mice, an adjuvant arthritis mouse model was established (0.01 mL of complete Freund's adjuvant (CFA, Sigma) was injected daily into the right hind toe of each mouse (Vital River, BALB/c), and one week later, an arthritis model was established followed by drug administration). Afterwards, the mice were administered by gavage (dose: 3 mg/mouse/day; note: Group 1A was CS, Group 1B was low-molecular CS that was CS obtained by the degradation method in Example 2 (having a weight-average molecular weight of 4.3k and having the same molecular weight distribution as that in Example 2), and Group 1C was CSX, which was the product of **Example 3**). After 30 days, blood was collected from the orbit to prepare serum. The IL-6 and IL-1β inflammatory factors were detected by Mouse IL-6 ELISA Kit (Art. No.: VAL604) and Mouse IL-1 ELISA Kit (R&D Systems, Art. No.: MLB00C).

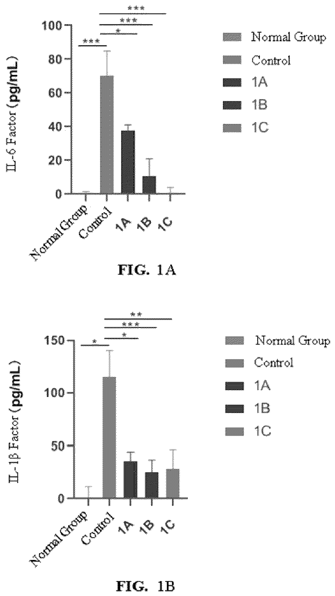

As shown in FIG. 1A and FIG. 1B, the results showed that the levels of IL-6 and IL-1β in the mouse serum were significantly reduced in Group 1C, the IL-6 and IL-1β levels in the serum were extremely low in the normal group (model-free group), and the IL-6 and IL-1β levels were significantly elevated in the control group (model group). The IL-6 level in the serum in Group 1C had already been comparable to that in the normal group (model-free group), and a strong inhibitory effect on inflammation was exhibited.

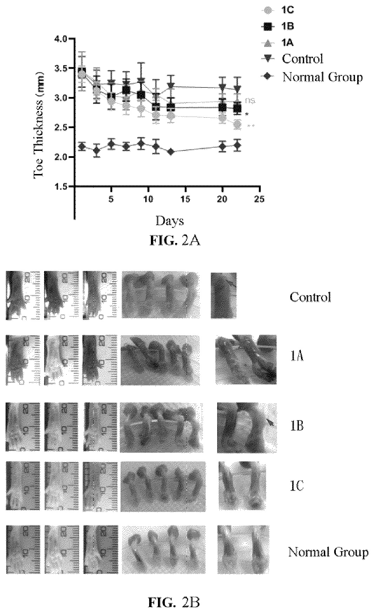

In addition, the width of the mouse toe was measured to analyze the level of swelling in toe. With reference to FIG. 2A, the results showed that as the administration time increased, the levels of swelling in mouse toes were significantly decreased in Groups 1B and 1C. In Group 1C, the swelling in toe had slowed down a lot at day 22 of administration, and several mice had already been similar to those in the normal group. Hence, Group 1C exhibited an effect of significantly reducing adjuvant-induced swelling in toe.

Anatomical analysis was conducted on the toes of mice. With reference to FIG. 2B, the results showed that significant redness, swelling and foci caused by significant inflammatory responses occurred to the legs in the control group (model group), and in Group 1C, no significant foci caused by significant inflammatory responses were observed, and there was no redness or swelling.

The results suggested that 3 mg/day/mouse of CSX could significantly ameliorate arthritis in mice, the effect of the low-molecular-weight CS came second, and the effect of conventional CS was the worst with inflammation still evident after 30 days.

### Test Example 2: Therapeutic Effects on Arthritis (Rheumatoid Arthritis)

In the rat rheumatoid arthritis model, CSX exhibited a significant therapeutic effect on rheumatoid arthritis with the following main manifestations: CSX could significantly improve the degrees of swelling in rat organs such as toes, liver, and spleen in the inflammation model, and reduce the inflammatory phenotypes of lymphocytes and neutrophils in the blood, and have an efficacy similar to that of methotrexate (MTX); CSX could basically cure injuries in joints and bones of foot caused by chronic inflammation, and had a better efficacy than that of MTX; and CSX did not have the toxic and side effects of MTX.

This test example analyzed the effect of CSX on rheumatoid arthritis in rats (Vital River, SD). Preparation of rat rheumatoid arthritis model: Bovine type II collagen (CII, Solarbio) was used to prepare a rat rheumatoid arthritis model. CII was mixed with an equal volume of complete Freund's adjuvant (CFA, Chondrex) to prepare an emulsion. The final CII concentration was 1.0 mg/mL in the emulsion. Six rats were randomly selected as the blank control group (*i.e.,* the normal group/blank group), and the rest of rats were used for preparation of the rheumatoid arthritis models. Except for the rats in the blank control group, all the rest of rats were injected with a total of 0.5 mL of emulsion at multiple sites - the back and the base of the tail. After 10 days, the same dose of an emulsion mixture of collagen and adjuvant was injected at the same sites of the first immunization to booster immunization. Grouping and administration: normal group (blank group), model group, positive control group (MTX, 0.2 mg/kg of methotrexate solution, administered by gavage every other day), and drug group (CSX obtained in **Example 2,** administered by gavage every day at a dose of 200 mg/kg).

The degrees of swelling in rat toes were observed at week 1, week 2, week 3, and week 4 after administration, and the toe thicknesses of the right hind limbs of rats in each group were measured and recorded. The specific method was as follows: after restricting the activities of rats, the right hind limbs of rats were gently straightened, the measured sites of the toes of rats were marked with a marker pen, and then the toe thicknesses were measured by a vernier caliper.

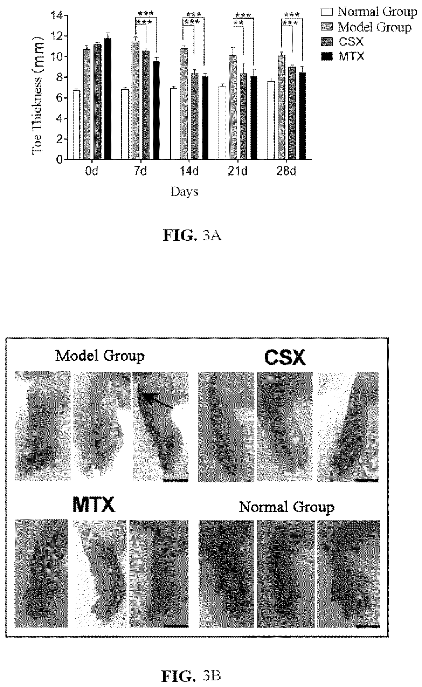

Referring to FIG. 3A and FIG. 3B, the results showed that the degrees of swelling in the toes of rats in the drug group (CSX) were decreased significantly at day 7 after administration without any adverse reaction; and the degrees of swelling in toes were further decreased at day 14 after administration. The toes of rats in the model group were always in a significant swelling state. The toe swelling remained at a relatively stable level after the drug administration was continued till day 28. The toe swelling did not show a further decline any more, which might be related to the hyperplasia of connective tissue caused by modeling of swelling. The rats in the positive control group (MTX) also showed the desired effect of inhibiting toe swelling, but accompanied with adverse reactions such as diarrhea and weight loss. The above results suggested that CSX had a significant inhibitory effect on toe swelling caused by rheumatoid arthritis in rats, and had no significant toxicity in rats.

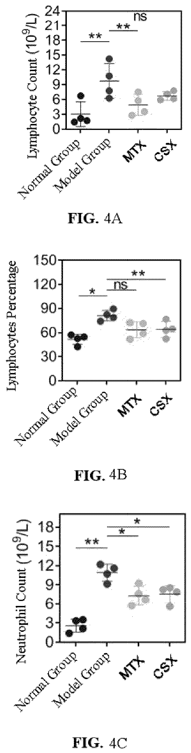

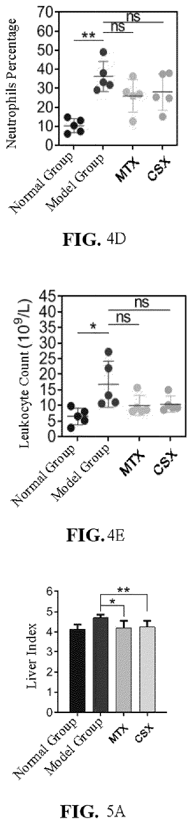

Referring to FIG. 4A to FIG. 4E, at day 14 after administration, venous blood of rats was collected and subjected to blood routine analysis. It could be observed that the absolute lymphocyte count in the positive control group (MTX) was significantly decreased as compared to the model group, and the lymphocytes percentage in the drug group (CSX) was significantly decreased as compared to the model group. Nonetheless, there was no statistical significant difference between the absolute lymphocyte counts in the drug group (CSX) and in the model group. Furthermore, the absolute neutrophil count in the positive control group (MTX) was significantly decreased as compared to the model group, so was that in the drug group (CSX). The absolute leukocyte count showed that there was no statistical difference between the model group and the drug group (CSX) as well as the positive control group (MTX).

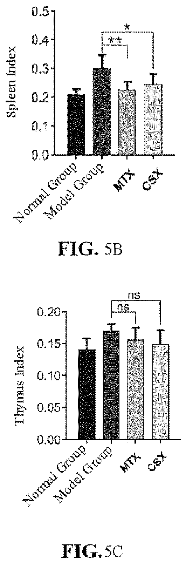

Referring to FIG. 5A to FIG. 5C, at day 28 after administration, the rats were dissected, and the liver, spleen, and thymus were removed and weighed accurately. Thereafter, the organ indices were determined based on the ratios of the organs to the rat weight. The results showed that CSX could significantly reduce the liver enlargement in rats. The spleen index reflected that CSX could significantly reduce the spleen enlargement in rats, suggesting a reduction in occurrence of chronic inflammation. There was no statistical significant difference in thymus index.

The above results suggested that CSX could significantly improve the degrees of swelling in toes, liver, spleen and the like of rats of the inflammation model, and reduce the inflammatory phenotypes of lymphocytes and neutrophils in the blood, and exhibit an efficacy similar to that of MTX.

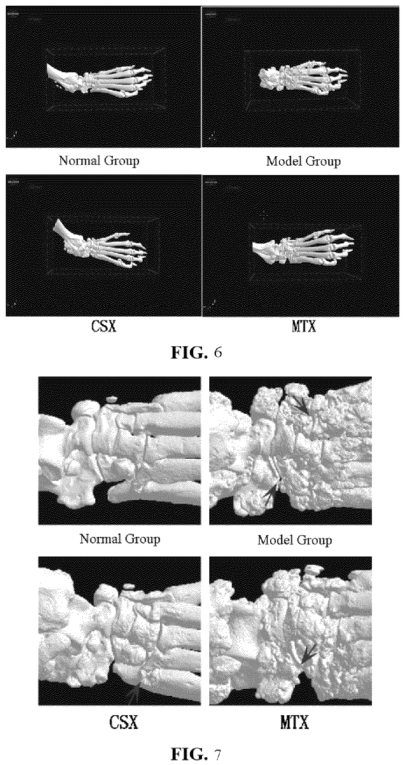

Referring to FIG. 6 and FIG. 7, the CT of ankle joint parts of rats showed the following results: In in the blank group (normal group), the interface of the rat joint part was clear, the junction site of joints was regular, and the surface of bones were smooth. In the model group, the binding interface of rat joints was unclear, the joints were irregular, the surface of bones was extremely unsmooth, and hyperplasia, swelling and the like occurred. In the drug group (CSX), there was a certain degree of damage to the joint parts of rats, but the interface of the joints was clearer than that in the model group, the joints were relatively regular, and the surface of bones was relatively smooth. In the positive control group (MTX), the surface of bones of the rat joints was relatively smooth as compared to the control group, and the binding interface of the joints was tighter than that in the model group, but the degree of joint injury was more serious than that in the rats of the drug group (CSX).

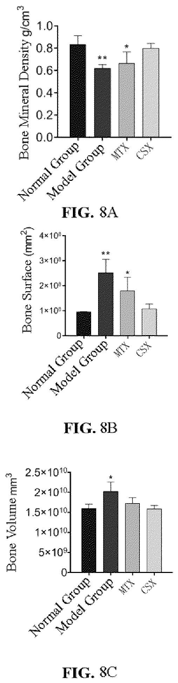

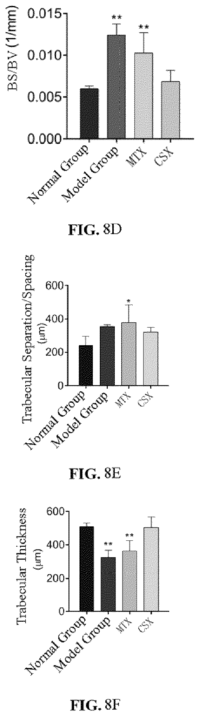

Referring to FIG. 8A to FIG. 8F, the CT results showed that the bone mineral density (BMD) in the model group was significantly lower than that in the blank group (normal group). After administration of CSX, the decrease in bone mineral density induced by inflammation could be effectively alleviated. The specific surface area of the ankle joint in the model group was significantly increased as compared to the blank group, but the bone surface (BS) of the ankle joint of rat decreased significantly after administration of CSX and MTX, with a more pronounced decrease in rats in the drug group (CSX). Rats in the drug group (CSX) exhibited a significant decrease in bone volume (BV) of the ankle joint and a significant decrease in the ratio of the bone surface to the bone volume (BS/BV). Trabecular Separation/Spacing (Tb.Sp) referred to the average width of the medullary cavity between trabeculae. Increased Tb.Sp might be associated with osteoporosis. In the model group, the Tb.Sp of rats increased significantly, but after administration of CSX, the Tb.Sp of rats decreased, suggesting that CSX exhibited an effect of reducing the osteoporosis induced by inflammation. Trabecular Thickness (Tb.Th) in the drug group (CSX) was somewhat elevated as compared to the model group, which also reflected that CSX could inhibit inflammation-induced osteoporosis. In summary, the CT results suggested that CSX had the effects of significantly inhibiting osteoporosis caused by inflammation, increasing the bone mineral density, and improving the smoothness of the surface of bones, and the effects of CSX were superior to those of MTX.

Also, the experiment showed that diarrhea appeared in the positive control group (MTX) at the dose administered in this experiment, while the drug group (CSX) did not show any apparent abnormalities. The acute toxicity test in mice showed that the drug group (CSX) did not show a toxic reaction at a single dose of up to 2000 mg/kg, suggesting that CSX might have better safety.

The above-mentioned CT results suggested that CSX was able to basically cure injuries of joints and bones of foot caused by chronic inflammation, and had a strong efficacy than that of MTX.

### Test Example 3: Blood Lipids-lowering Activity

To evaluate the effect of CSX on lowering blood lipids, this test example evaluated the levels of total cholesterol (T-CHO), triglyceride (TG), and high/low density lipoprotein cholesterol (H/LDL-C) in C57/b6 mice by feeding them with high-fat diets (60 kcal% fat (Research diets)) for three weeks, and then administered by gavage for 43 days (note: Group 2A was ordinary CS, Group 2B was low-molecular CS that was the CS obtained by the degradation method in Example 2 (having a weight-average molecular weight of 4.3k and having the same molecular weight distribution as that in Example 2), and Group 2C was Extract A-3 product CSX obtained in **Example 1,** all administered by gavage at a dose of 6 mg/mouse daily). The blood lipid-free model group was a group fed with normal diets; the blood lipid model group was fed with high-fat diets, but the group administered by gavage with PBS alone and the normal diet group were switched to the groups fed with normal diets three weeks later after modelling. Ordinary chondroitin was the bovine-derived chondroitin provided by Hunan Wuxing Biological Technology Co., Ltd., and had a weight-average molecular weight of 21k, among which the proportion of chondroitin having a molecular weight of greater than 50000 was 0%, the proportion of chondroitin having a molecular weight of 25000 to 50000 was 31%, the proportion of chondroitin having a molecular weight of 400 to 25000 was 69%, and the proportion of chondroitin having a molecular weight of 400 or less was 0%.

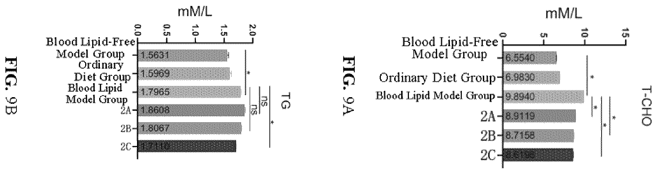

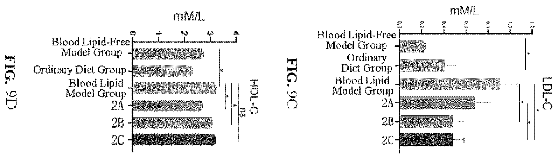

Referring to FIG. 9A to FIG. 9D, the total cholesterol levels in Groups 2A, 2B, and 2C were all significantly reduced as compared to the blood lipid model group, with the most pronounced reduction in Group 2C. The total triglycerides in Groups 2A and 2B were not significantly reduced as compared to the blood lipid model group, while Group 2C exhibited a significant reduction. The low density lipoprotein cholesterols in Groups 2A, 2B, and 2C were significantly reduced as compared to the blood lipid model group, with the most pronounced reduction in Group 2C. The high density lipoprotein cholesterols in Groups 2A and 2B exhibited a significant reduction but no significant reduction in Group 2C, as compared to the blood lipid model group. The animal experiment in this test example suggested that CSX exhibited the effect of significantly lowering the serum total cholesterol, serum total triglycerides, and low density lipoprotein.

### Test Example 4: Activity against Skin Inflammation

To evaluate the anti-inflammatory effect of CSX on skin inflammation, this test example used a mouse ear swelling model, and sensitized the right ear of the mouse three times. The sensitizer was phorbol ester (Psaitong reagent, China) at a concentration of 0.125 mg/mL. The sensitization was conducted by the method of smearing the sensitizer on the right ear of a mouse, 20 µL for each time (10 µL per side) at an interval of 48 h between the sensitizations. Group 4A was an aqueous solution of CSX (product obtained in **Example 3**). Group 4B was an aqueous compound solution of low-molecular CS and spermidine (the molar mass ratio of spermidine was the same as the molar ratio of polyamines in Group 4A, the molar weight of spermidine was the same as the total molar weight of the polyamines contained in the extract of Example 3; the amount of CS was the same as the mass of the extract minus polyamines, and the low-molecular CS was CS obtained by the degradation method in Example 2 (having a weight-average molecular weight of 4.3k and having the same molecular weight distribution as that in Example 2)). Group 4C was a negative control group (ultrapure water). Group 4D was an aqueous compound solution of ordinary CS and spermidine (the molar mass ratio of spermidine was the same as the molar ratio of polyamine in Group 4A, *i.e.,* the molar weight of spermidine was the same as the total molar weight of the polyamines contained in the extract of Example 3; the amount of CS was the same as the mass of the extract minus polyamines). The ordinary CS was the chondroitin provided by Hunan Wuxing Biological Technology Co., Ltd., which had a weight-average molecular weight of 48k, among which the content of chondroitin having a molecular weight of greater than 50000 was 34%, the content at 25000 to 50000 was 55%, and the content at 400 to 25000 was 11%. In Groups 4A, 4B and 4D, the concentrations of the solutions were all 2 w/v%, the drug was administered once daily for a total of 6 consecutive days by smearing it on the surface at a dose of 20 µL/mouse in all groups. The evaluation indexes were primarily the degree of swelling and body weight of the mouse ear. The degree of swelling in mouse ear was evaluated by the method of sacrificing the a mouse by cervical dislocation, cutting off both ears, taking left and right symmetrical ear pieces with a perforator 6 mm in diameter, weighing them on an electronic balance, and calculating the weight difference between the left and right ears as the degree of swelling.

The results were shown in Table 2 and Table 3 below. It followed that both the product CSX of Example 3 and the complex of spermidine and low-molecular CS exhibited a significant effect of eliminating inflammation, and the product of Example 3 exhibited a better effect, but both were significantly superior to that in the negative control group. Moreover, CSX had no effect on the body weight of mice and no obvious side effects were found. The effect of the complex of low-molecular CS and spermidine was better than the effect of the complex of ordinary CS and spermidine.

**Table 2. Statistics of Degree of Swelling in Mouse Ear**

| | No. | Left Ear Weight (mg) | Right Ear Weight (mg) | Degree of Swelling | Average Degree of Swelling |
|---|---|---|---|---|---|
| 4A | 1 | 7.33 | 14.22 | 6.89 | 6.74 |
| | 2 | 9.45 | 15.51 | 6.06 | |
| | 3 | 8.28 | 15.56 | 7.28 | |
| 4B | 4 | 8.71 | 17.93 | 9.22 | 9.25 |
| | 5 | 7.09 | 15.40 | 8.31 | |
| | 6 | 7.55 | 17.77 | 10.22 | |
| 4C | 7 | 6.86 | 17.42 | 10.55 | 13.20 |
| | 8 | 7.34 | 21.56 | 14.22 | |
| | 9 | 7.57 | 22.41 | 14.84 | |
| 4D | 10 | 8.91 | 18.62 | 9.71 | 11.02 |
| | 11 | 7.59 | 18.87 | 11.31 | |
| | 12 | 8.16 | 20.22 | 12.06 | |

**Table 3. Statistics of Body Weight of Mice**

| | No. | Starting Weight (mg) | Final Weight (mg) | Difference | Mean Difference |
|---|---|---|---|---|---|
| 4A | 1 | 22.68 | 22.30 | -0.38 | 0.20 |
| | 2 | 22.17 | 22.12 | -0.05 | |
| | 3 | 20.36 | 21.40 | 1.04 | |
| 4B | 4 | 22.02 | 22.36 | 0.34 | 0.28 |
| | 5 | 23.75 | 22.85 | -0.9 | |
| | 6 | 19.80 | 21.20 | 1.4 | |
| 4C | 7 | 22.28 | 21.76 | -0.52 | 0.22 |
| | 8 | 21.41 | 22.35 | 0.11 | |
| | 9 | 22.43 | 23.06 | 0.63 | |
| 4D | 10 | 20.89 | 21.02 | 0.13 | 0.17 |
| | 11 | 21.44 | 21.64 | 0.20 | |
| | 12 | 20.44 | 20.62 | 0.18 | |

### Test Example 5: Antioxidant Activity

To evaluate the antioxidant effect of CSX, human liver HEPG2 cells were used as a model in this test example to test the effect of CSX on cellular reactive oxygen species by the fluorescent probe method. In this test example, CSX-1 was Extract A-3 product obtained in Example 1. CSX-2 was a complex of low-molecular CS and polyamines (spermine, spermidine, and putrescine) at the same compounding ratio as that of CSX-1 (the ratio of the three polyamines was the same as that in Extract A-3 of Example 1, the total molar weight of polyamines was the same as that of the polyamines contained in Extract A-3 of Example 1; the amount of CS was the same as the mass of the extract minus polyamines), and the low-molecular CS was CS obtained by the degradation method described in Example 2 (having a weight-average molecular weight of 4.3k and having the same molecular weight distribution as that in Example 2).

This experiment followed the principle of conducting a reactive oxygen species assay by the fluorescent probe DCFH-DA. DCFH-DA itself was not fluorescent and could freely pass through the cell membrane. After entry into the cell, it could be hydrolyzed by esterases in the cell to produce DCFH. However, DCFH was unable to pass through the cell membrane, thereby allowing the probe to be easily loaded into the cell. The reactive oxygen species in the cell could oxidize non-fluorescent DCFH to produce fluorescent DCF. The level of intracellular reactive oxygen species could be determined by detecting the fluorescence of DCF.

Cells in logarithmic growth phase were taken and the cell state was observed under a microscope. The cells were washed twice with sterile PBS. The supernatant was discarded. 3 mL of PBS was added, and 0.5 mL of 2.5% trypsin was added and digested at 37°C for 5 min. 3 mL of serum-containing medium was added to terminate the reaction. The resultant was aspirated into a 15-mL centrifuge tube, and centrifuged at 1000 rpm. The supernatant was discarded. An appropriate amount of medium was added. The cells were counted. 2000 to 10000 cells were counted and placed in each well of a 96-well plate, cultured at 37°C for 6 h to allow the cells adhered to the wall, and stimulated by adding drugs at different concentration. After 24 h, the plate was removed. DCFH-DA was diluted with serum-free medium at 1: 1000, so that the final probe concentration was 10 µM. The cell medium was removed, and an appropriate volume of DCFH-DA that had been diluted was added. The cells were incubated in a cell culture incubator at 37°C for 20 min. The cells were washed three times with the serum-free cell culture solution, so as to completely remove the DCFH-DA that had not entered the cells. The intensity of fluorescence before and after stimulation was detected by flow cytometry at an excitation wavelength of 488 nm and an emission wavelength of 525 nm.

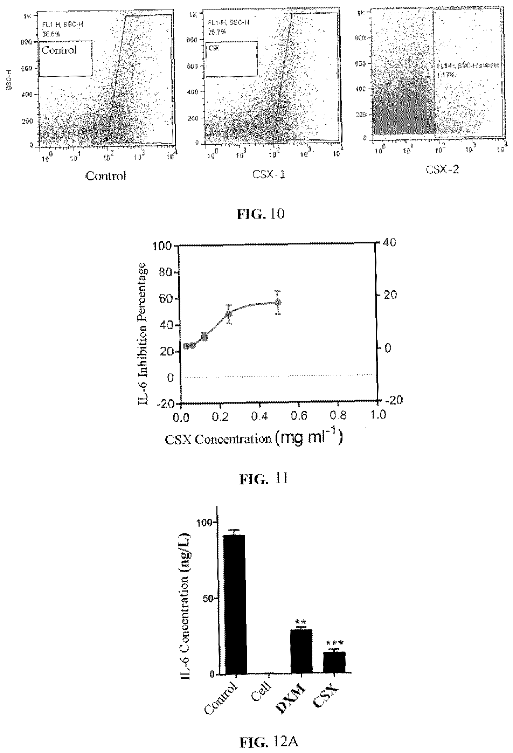

The cytological experiment suggested that CSX (especially low-molecular CSX) exhibited a significant antioxidant capacity at a final concentration of 0.25 mg/mL. Compared with the control group (untreated cells), the number of reactive oxygen species (ROS)-positive cells in the CSX-1 group dropped from 36.5% to 25.7%, and the number in CSX-2 further dropped to 1.17% (FIG. 10).

### Test Example 6: Test on Activity against Alzheimer's Disease-Related Inflammation

Studies on neurodegenerative diseases such as Alzheimer's disease (AD) showed that the inflammatory process played a fundamental role in the pathogenesis of AD, and inflammatory responses mediated by microglia in the central nervous system were one of the leading causes of neurodegenerative diseases, especially Alzheimer's disease. The inflammatory components associated with AD included brain cells such as microglia and astrocytes. IL-6 released by microglia was considered to be one of the major factors in their mediation of the occurrence of AD.

To detect the inhibitory effect of CSX on microglia inflammation, human microglia HMC3 were treated with CSX (prepared in **Example 2**). The results showed that CSX could significantly inhibit the expression level of IL-6 in microglia induced by LPS (FIG. 11).

### Test Example 7: Inhibitory Effect of CSX on Inflammatory Responses at Cellular Level

Induced by lipopolysaccharide (LPS), the mouse monocyte RAW264.7 would lead to up-regulated expression levels of many inflammatory factors in the cells, such as IL-6, TNF-α, and IL-1β. This test example analyzed whether CSX could inhibit the production of cellular inflammatory factors. The CSX used in this test example was the product of Example 3.

To the DMEM 10% FBS (Life technology) medium, LPS (Solarbio) was added at a final concentration of 0.1 µg/mL. LPS-containing 10% FBS DMEM was used to formulate CSX at a final concentration of 0.6 mg/mL, 100 µl of which was added to each well. There were three replicates per gradient. The Dexamethasone (DXM) stock solution was used at an amount of 2 mg/mL, and subjected to 1000-fold dilution with LPS-containing 10%FBS DMEM, 100 µl of which was added to each well. There were three replicates per gradient. An LPS stimulus-free cell group and an LPS stimulated cell group were set. Three replicates were set for each group. After culturing for 24 h, the supernatant was aspirated cautiously and used directly for the enzyme linked immunosorbent assay (ELISA).

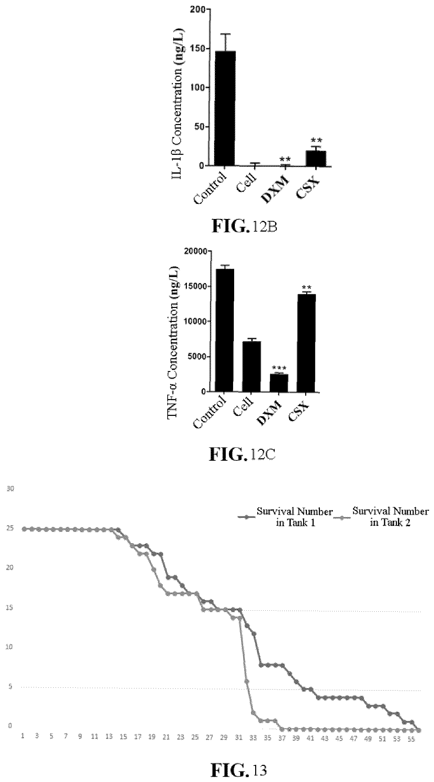

Referring to FIG. 12A to FIG. 12C, the results showed that CSX could significantly inhibit the expression of IL-6, IL-1β, and TNF-α in mouse mononuclear macrophages induced by LPS. The inhibitory effect of CSX on IL-6 was better than that of Dexamethasone. This proved that CSX exhibited a very good inhibitory effect on the expression levels of inflammatory factors at the cellular level.

### Test Example 8: Aging-Delay/Lifespan-Extension Activities

To evaluate the lifespan-extending activity of CSX, guppies (*Poecilia* reticulate) were used as the research object to detect the survival time of offspring. The experimental fish was a captive-bred pregnant guppy (trade name: "Lucky Strike") purchased from Shilihe Bird, Flower, Fish, and Insect Market in Beijing. After one week of breeding, the experimental fish produced more than 50 fry, and after 3 days of stable breeding, the fry were divided into two groups for breeding, with 25 fry in each group. The CSX used in this test example was the product of **Example 3.**

Feeding conditions: Each tank contained about 10L of water that had been air-dried for one week, maintained at a constant temperature of 26°C, and equipped with a water circulation filtration system and an oxygenation device. The water was replenished to 10L every two days, and about 1/3 of the water (about 3L to 4L) was replaced every week. The water was bottled drinking water. The fry were fed with an appropriate amount of juvenile fish diets once a day. Tank 1 added with CSX: 30 mg/L of CSX was added after water exchange every week; and Tank 2 with fry cultivated normally: the water was replaced every week without adding CSX. The number of fry deaths was observed and counted every day, and the average survival time of fry in each tank was calculated by the sum of (days of survival * number of fry)/total number of fry.

The results showed that the average survival time in Tank 1 was 31.88 days, and the average survival time in Tank 2 was 27.48 days, indicating that 30 mg/L of CSX could extend the survival time of fry of captive-bred guppy by 16.01%. The results were shown in FIG. 13.

To further evaluate the aging-delay/lifespan-extension activity of CSX, *Drosophila melanogaster* (raised in our laboratory) was used as the research object to detect its survival time. The experimental *Drosophila melanogaster* was the w1118 wild-type *Drosophila melanogaster* raised in the laboratory, and the basal diet was formulated as a liquid diet of 2.2% of sucrose, 8% of malt extract, 1.8% of yeast, and 1.2% of butyl paraben. The female and male *Drosophilae* were fed with three different formulas of diets. Formula 1 was the basal diet formula; Formula 2 was the basal diet formula added with 0.1 g/L of CSX (the product of Example 2); and Formula 3 was the basal diet formula added with a combination of spermidine hydrochloride and spermine hydrochloride (the molar weight was 10 times that of the polyamines in formula 2). Six groups were set for each formula, including 3 groups of female *Drosophilae* and 3 groups of male *Drosophilae,* with 20 *Drosophilae* in each group, all of which were female or male *Drosophilae* that had not mated within 4 hours after eclosion. The number of *Drosophila* deaths was observed and counted daily, and 400 µl of the corresponding diets were supplemented to each group once a day. The average survival time of *Drosophilae* in each group was calculated by the sum of (days of survival * number of *Drosophilae*)/total number of *Drosophilae,* respectively.

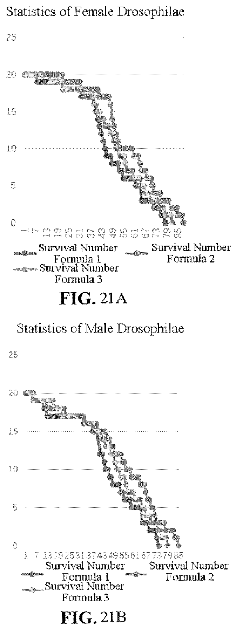

Referring to FIG. 21A to FIG. 21B, the results showed that the average survival time of *Drosophilae* raised with Formula 1 was 46.2 to 49 days; the average survival time of *Drosophilae* raised with Formula 2 was 54.5 to 59 days; and the average survival time of *Drosophilae* raised with Formula 3 was 50.6 to 52.5 days. This suggested that compared with ordinary diets, CSX could extend the lifespan of *Drosophilae* by 18.0% to 20.4%. It was worth noting that the total molar weight of polyamines in CSX of Formula 2 was only one-tenth of that of the free polyamines in Formula 3, but the lifespan-extension effect in the CSX group was 7.7% to 12.38% higher than that of ordinary polyamines.

### Test Example 9: Test on Anti-Inflammatory Activity

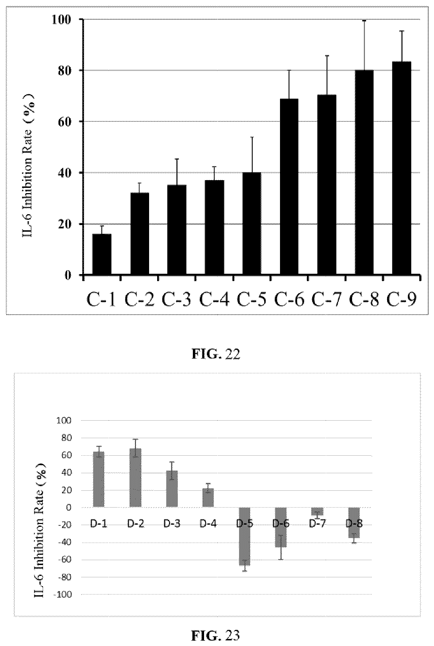

In this test example, the anti-inflammatory activities of various substances against IL-6 were tested by the same detection method of Example 1. All the low-molecular CS used in this test example were the CS (having a weight-average molecular weight of 4.3k and having the same molecular weight distribution as that in Example 2) obtained by the degradation method in Example 2. Of these, C-1 was low-molecular CS; C-2 was spermine; C-3 was spermidine; C-4 was spermidine + spermine; C-5 was spermidine + spermine + putrescine; C-6 was low-molecular CS + spermine; C-7 was low-molecular CS + spermidine; C-8 was low-molecular CS + spermidine + spermine; and C-9 was low-molecular CS + spermidine + spermine + putrescine. The total molar weight of polyamines in C3 to C12 was the same, and the molar ratio of each of polyamines was the same where two or more polyamines were involved. As was clear from the above results, the activity of the complex of CS and polyamine was significantly superior to that of a single low-molecular CS or polyamine, and the effect of a combination of multiple amines was superior to that of a single amine (FIG. 22).

### Test Example 10: Comparison of Activities of Low-Molecular CS with Biogenic Polyamine and Abiogenic Polyamine

In this test example, the anti-inflammatory activities of combinations of low-molecular CS with various biogenic polyamines on IL-6 were tested by the detection method of Example 1. All the low-molecular CS were CS (having a weight-average molecular weight of 4.3k and having the same molecular weight distribution as that in Example 2) obtained by the degradation method in Example 2. All the low-molecular CS used in this test example were CS (having a weight-average molecular weight of 4.3k and having the same molecular weight distribution as that in Example 2) obtained by the degradation method in Example 2. The results were shown in FIG. 23. D-1 was low-molecular CS + spermine; D-2 was low-molecular CS + spermidine; D-3 was low-molecular CS + putrescine; D-4 was low-molecular CS + cadaverin; D-5 was low-molecular CS + histamine; D-6 was low-molecular CS + tryptamine; D-7 was low-molecular CS + 1,7-diaminoheptane; and D-8 was low-molecular CS + pentaethylenehexamine. The total molar concentration-mass ratio of polyamines in each group was the same at 2 µmol/g, and the molar ratio of each of polyamines was the same where two or more polyamines were involved. As was clear from the above results, the activities of the combinations of low-molecular CS with various polyamines were different, among which its activity with spermine was comparable to that with spermidine, and superior to that with putrescine, more superior to that with cadaverin. Histamine and tryptamine as well as abiogenic polyamines 1,7-diaminoheptane and pentaethylenehexamine had inflammatory effects, and histamine had the strongest effect.

### Test Example 11: Comparison of Activities of Chondroitin Sulfate Biogenic Polyamine Complexes Containing Different Contents of Biogenic Polyamines

In this test example, low-molecular CS was compounded with spermine at different ratios, and the stability of the compound combinations was tested. All the low-molecular CS were CS (having a weight-average molecular weight of 4.3k and having the same molecular weight distribution as that in Example 2) obtained by the degradation method in Example 2. The experimental method was as follows: 40 mg/ml of aqueous solution of low-molecular CS was formulated, and an aqueous solution of spermine hydrochloride was formulated according to the concentration listed in Table 4 below. Two solutions at the same volume were measured and mixed well to yield compound solutions E-1 to E-8. The transmittance of the solutions was determined using a UV spectrophotometer (wavelength: 600 nm). Among them, the transmittance of E-7 and E-8 was significantly reduced, probably due to the formation of colloidal solutions at the nanometer to micrometre scale. The stability of the compound solutions was determined by two approaches: 1. the compound solutions were left to stand for 7 days and their stability was observed; and 2. 20 µl of the compound solution was added to 1 ml of rabbit plasma solution (Beijing Land Bridge), and the changes in the solution were observed to simulate its stability in blood. The results were shown in Table 4 below. As could be appreciated from the above results, if the spermine content was too high (E-7 and E-8), the spermine was prone to interacting with chondroitin to produce a precipitate, rendering the system unstable. Furthermore, if the spermine content was too high (E-6, E-7, and E-8), the spermine was prone to electrostatic binding with serum proteins, resulting in a large amount of precipitation, and its cytotoxicity was found to be remarkable in cellular experiments. This suggested that the combination with too high content of spermine exhibited poor stability and high toxicity, and did not have practical application value. E-6, E-7, and E-8 were synthesized by the method of Cited Literature 4.

**Table 4:**

| No. | Spermine Concentration (mg/ml) | Molar-Mass Ratio of Biogenic Polyamine in CSX (µmol/g) | Transmittance | 7-Day Standing Stability | Stability in Plasma Solution | Cytotoxicity | IL-6 Inhibition Rate |
|---|---|---|---|---|---|---|---|
| E-1 | 0.003 | 0.22 | 99% | No precipitation | No precipitation | None | 22% |
| E-2 | 0.007 | 0.50 | 99% | No precipitation | No precipitation | None | 61% |
| E-3 | 0.027 | 1.94 | 99% | No precipitation | No precipitation | None | 70% |
| E-4 | 0.273 | 19.60 | 99% | No precipitation | No precipitation | None | 84% |
| E-5 | 2.73 | 196.01 | 96% | No precipitation | Almost no precipitation | None | 92% |
| E-6 | 5.5 | 394.89 | 91% | No precipitation | Lot of precipitation | Medium | -* |
| E-7 | 14 | 1005.17 | 62% | Precipitation | Lot of precipitation | High | -* |
| E-8 | 55 | 3948.88 | 34% | Precipitation | Lot of precipitation | High | -* |

| | | | | | | | |
|---|---|---|---|---|---|---|---|
| Note: "-*" indicated no valid data on the IL-6 inhibition rate due to excessive cytotoxicity. | | | | | | | |

### Test Example 12: Demonstration That Effects of Other Polysaccharides and CS Having Other Weight-Average Molecular Weights Were Not As Effective As

### Combinations of the Present Disclosure

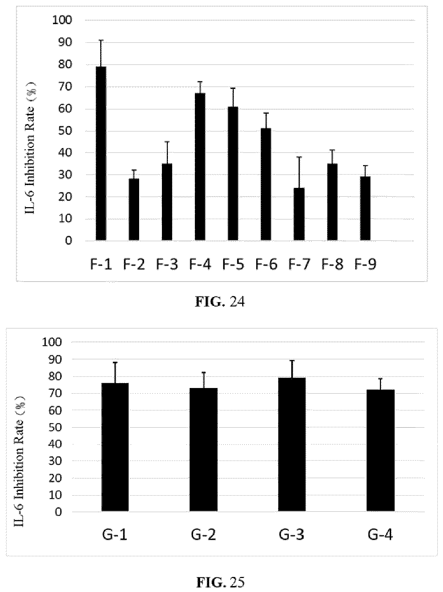

In this test example, the anti-inflammatory activities of combinations of CS having various molecular weight distributions and other anionic polysaccharides with polyamines against IL-6 were tested by the same detection method of Example 1. In the combinations, the polyamine was a combination of putrescine, spermine, and spermidine, where the total molar concentration-mass ratio of polyamines was the same at 2 µmol/g, and the molar ratio of each of polyamines was the same where two or more polyamines were involved. F-1 was CS + polyamine, which was CS obtained by the degradation method in Example 2 that had a weight-average molecular weight of 4.3k and had the same molecular weight distribution as that in Example 2. F-2 was CS + polyamine, in which the CS was the product provided by Hunan Wuxing Biological Technology Co., Ltd. that had a weight-average molecular weight of 48k, among which the content of CS having a molecular weight of greater than 50000 was 34%, the content at 25000 to 50000 was 55%, and the content at 400 to 25000 was 11%. F-3 was CS + polyamine, in which the CS was the product provided by Hunan Wuxing Biological Technology Co., Ltd. that had a weight-average molecular weight of 35k, among which the content of CS having a molecular weight of greater than 50000 was 12%, the content at 25000 to 50000 was 68%, and the content at 400 to 25000 was 20%. F-4 was CS + polyamine, in which the CS was the product provided by Hunan Wuxing Biological Technology Co., Ltd. that was prepared by the enzymolysis method and had a weight-average molecular weight of 879, with the proportion at 400 to 2000 being 99%, among which the proportion at 400 to 1000 was 54%, the proportion at 1000 to 2000 was 45%, the proportion at less than 400 was 1%, and the proportion at greater than 2000 was 0%. F-5 was CS + polyamine, in which the CS was the product provided by Hunan Wuxing Biological Technology Co., Ltd. that had a weight-average molecular weight of 27k, among which the content of CS having a molecular weight of greater than 50000 was 0%, the content at 25000 to 50000 was 53%, and the content at 400 to 25000 was 47%. F-6 was CS + polyamine, in which the CS was the product provided by Hunan Wuxing Biological Technology Co., Ltd. that was prepared by the enzymolysis method and had a weight-average molecular weight of 598, where the proportion at less than 400 was 11%, the proportion at greater than 2000 was 0%, and the proportion at 400 to 2000 was 89%, among which the proportion at 400 to 1000 was 85% and the proportion at 1000 to 2000 was 4%. F-7 was complete CS hydrolysate + polyamine, where the complete CS hydrolysate had a weight-average molecular weight of 415, among which the proportion at less than 400 was 56% and the proportion at greater than 400 was 44%. The complete CS hydrolysate was prepared by hydrolyzing chondroitin sulfate in 6 M hydrochloric acid at 100°C for 4 h. F-8 was hyaluronic acid + polyamine. F-9 was trehalose + polyamine. The test results were shown in FIG. 24. As could be appreciated from the above results, the activities of complexes F-1, F-4, F-5, and F-6 of low-molecular CS and polyamines were significantly superior to those of the combinations of CS having other molecular weight distributions or other anionic polysaccharides with polyamines.

### Test Example 13: Demonstration That Various Molar Ratios of Polyamines Had No Significant Effects on Activity

In this test example, the anti-inflammatory activities of combinations of CS with polyamines at different molar ratios against IL-6 were tested by the same detection method of Example 1. All the CS were CS (having a weight-average molecular weight of 4.3k and having the same molecular weight distribution as that in Example 2) obtained by the degradation method in Example 2. In the combinations, the polyamine was a combination of putrescine, spermine, and spermidine, where the total molar concentration-mass ratio of polyamines was the same at 2 µmol/g. The molar ratio of polyamines in G-1 was spermine: spermidine: putrescine = 1:1:1; the molar ratio of polyamines in G-2 was spermine: spermidine: putrescine = 5:1:1; the molar ratio of polyamines in G-3 was spermine: spermidine: putrescine = 1:5:1; and the molar ratio of polyamines in G-4 was spermine: spermidine: putrescine = 1:1:5. The test results were shown in FIG. 25. As could be appreciated from the above results, when the total molar weight of polyamines was the same in the case of the same molecular weight and molecular weight distribution of CS, different molar ratios of the polyamines would not lead to a significant effect on the activity.

## Claims

1. A chondroitin sulfate biogenic polyamine complex, which is a complex of chondroitin sulfate and biogenic polyamine, wherein the chondroitin sulfate is non-covalently linked to the biogenic polyamine, and the biogenic polyamine comprises one or a combination of two or a combination of three or more of spermine, spermidine, putrescine, and cadaverine.

2. The chondroitin sulfate biogenic polyamine complex according to claim 1, wherein the chondroitin sulfate is chondroitin sulfate in an acid form or chondroitin sulfate in a salt form.

3. The chondroitin sulfate biogenic polyamine complex according to claim 1 or 2, wherein
among the chondroitin sulfate, according to a GPC integral ratio, a proportion of chondroitin sulfate having a weight-average molecular weight of 50000 or more is 0%; and
among the chondroitin sulfate, according to the GPC integral ratio, a proportion of chondroitin sulfate having a weight-average molecular weight of 25000 to 50000 is 40% or less;
preferably, the proportion of the chondroitin sulfate having a weight-average molecular weight of 25000 to 50000 is 35% or less;
further preferably, the proportion of the chondroitin sulfate having a weight-average molecular weight of 25000 to 50000 is 30% or less, 29% or less, 28% or less, 27% or less, 26% or less, 25% or less, 24% or less, 23% or less, 22% or less, 21% or less, 20% or less, 19% or less, 18% or less, 17% or less, 16% or less, 15% or less, 14% or less, 13% or less, 12% or less, 11% or less, 10% or less, 9% or less, 8% or less, 7% or less, 6% or less, 5% or less, 4% or less, 3% or less, 2% or less, 1% or less, or 0%; and
among the chondroitin sulfate, according to the GPC integral ratio, an upper limit of a proportion of chondroitin sulfate having a weight-average molecular weight of 400 to 25000 is 80% or more, preferably 81%, 82%, 83%, 84%, 85%, 86%, 87%, 88%, 89%, 90%, 91%, 92%, 93%, 94%, 95%, 96%, 97%, 98%, 99% or more, or 100%; and
a lower limit of the proportion of the chondroitin sulfate having a weight-average molecular weight of 400 to 25000 is 40% or more, preferably 41%, 42%, 43%, 44%, 45%, 46%, 47%, 48%, 49%, 50%, 51%, 52%, 53%, 54%, 55%, 56%, 57%, 58%, 59%, or 60% or more; and
among the chondroitin sulfate, according to the GPC integral ratio, a proportion of chondroitin sulfate having a weight-average molecular weight of 400 or less is 15% or less, preferably 3% or less, more preferably 1% or less, most preferably 0%; and/or
a proportion of chondroitin sulfate having an upper limit of the weight-average molecular weight of 25000 or less, preferably 24000, 23000, 22000, 21000, 20000, 19000, 18000, 17000, 16000, 15000, 14000, 13000, 12000, 11000, 10000, 9000, or 8000 or less and a lower limit of the weight-average molecular weight of 400, 500, 600, 700, or 800 or more has an upper limit of 80% or more, preferably 81%, 82%, 83%, 84%, 85%, 86%, 87%, 88%, 89%, 90%, 91%, 92%, 93%, 94%, 95%, 96%, 97%, 98%, 99% or more, or 100%, and has a lower limit of 40% or more, preferably 41%, 42%, 43%, 44%, 45%, 46%, 47%, 48%, 49%, 50%, 51%, 52%, 53%, 54%, 55%, 56%, 57%, 58%, 59%, or 60% or more;
wherein a sum of the proportion of the chondroitin sulfate having a weight-average molecular weight of 25000 to 50000, the proportion of the chondroitin sulfate having a weight-average molecular weight of 400 to 25000, and the proportion of the chondroitin sulfate having a weight-average molecular weight of 400 or less is 100%.

4. The chondroitin sulfate biogenic polyamine complex according to any one of claims 1 to 3, wherein among the chondroitin sulfate, according to a GPC integral ratio, a proportion of chondroitin sulfate having a weight-average molecular weight of 400 to 10000 is 40% to 100%, preferably 50% to 100%, 60% to 100%, 70% to 100%, or 80% to 100%;
preferably, according to the GPC integral ratio, the chondroitin sulfate has the following molecular weight distribution, in which the proportion of chondroitin sulfate having a weight-average molecular weight of greater than 50000 is 0%, the proportion of chondroitin sulfate having a weight-average molecular weight of 25000 to 50000 is 0% to 40%, the proportion of chondroitin sulfate having a weight-average molecular weight of 400 to 10000 and preferably 400 to 8000 is 40% to 100%, and the proportion of chondroitin sulfate having a weight-average molecular weight of 400 or less is 15% or less.

5. The chondroitin sulfate biogenic polyamine complex according to any one of claims 1 to 4, wherein the chondroitin sulfate biogenic polyamine complex has a weight-average molecular weight of 400 to 50000,
preferably, the chondroitin sulfate biogenic polyamine complex has a weight-average molecular weight of 400 to 25000.

6. The chondroitin sulfate biogenic polyamine complex according to any one of claims 1 to 5, wherein based on a total weight (g) of the chondroitin sulfate biogenic polyamine complex, the chondroitin sulfate biogenic polyamine complex contains 200 µmol/g or less, preferably 199 µmol/g or less, 198 µmol/g or less, 197 µmol/g or less, 196 µmol/g or less, 195 µmol/g or less, 194 µmol/g or less, 193 µmol/g or less, 192 µmol/g or less, 191 µmol/g or less, 190 µmol/g or less, 189 µmol/g or less, 188 µmol/g or less, 187 µmol/g or less, 186 µmol/g or less, 185 µmol/g or less, 184 µmol/g or less, 183 µmol/g or less, 182 µmol/g or less, 181 µmol/g or less, or 180 µmol/g or less of the biogenic polyamine; and
the chondroitin sulfate biogenic polyamine complex contains 0.5 µmol/g or more, preferably 0.6 µmol/g or more, 0.7 µmol/g or more, 0.8 µmol/g or more, 0.9 µmol/g or more, or 1 µmol/g or more of the biogenic polyamine.

7. The chondroitin sulfate biogenic polyamine complex according to any one of claims 1 to 6, wherein a mass percentage of protein in the chondroitin sulfate biogenic polyamine complex is less than 8%, preferably less than 5%, more preferably less than 3%, most preferably less than 1%, and further preferably 0%.

8. A preparation method of the chondroitin sulfate biogenic polyamine complex according to any one of claims 1 to 7, wherein the preparation method comprises a step of mixing chondroitin sulfate and biogenic polyamine, wherein based on a total weight (g) of the chondroitin sulfate biogenic polyamine complex, the chondroitin sulfate biogenic polyamine complex contains 0.5 to 200 µmol/g of the biogenic polyamine.

9. A preparation method of the chondroitin sulfate biogenic polyamine complex according to any one of claims 1 to 7, wherein the preparation method comprises a step of mixing an extraction solution containing chondroitin sulfate and polyamine separated and extracted from a raw material with ethanol, wherein the extraction solution has a pH value of 4 to 6, and a volume ratio of the extraction solution to ethanol is 1:1 to 1:3.

10. The preparation method according to claim 9, wherein separating and extracting the chondroitin sulfate and the polyamine from the raw material comprises:
a step of enzymolysis or acidolysis, wherein the raw material is lysed by enzymolysis or acidolysis to obtain an enzymatic hydrolysate or an acid hydrolysate; and
a step of separating and extracting the chondroitin sulfate and the polyamine, wherein the chondroitin sulfate and the polyamine are extracted simultaneously or stepwise from the enzymatic hydrolysate or the acid hydrolysate.

11. The preparation method according to claim 10, wherein, for stepwise extraction of the chondroitin sulfate and the polyamine, the polyamine is separated from the enzymatic hydrolysate or the acid hydrolysate by chromatography or extraction, and after the polyamine is separated, a residue is treated by one or more of enzymolysis, protein precipitation method, chromatography, and alcohol precipitation method to separate the chondroitin sulfate from the residue;
alternatively, a step of depolymerizing the chondroitin sulfate is further included after the chondroitin sulfate is separated.

12. The preparation method according to claim 10, wherein, for simultaneous extraction of the chondroitin sulfate and the polyamine, a protein precipitation method is adopted to precipitate a protein in the enzymatic hydrolysate or the acid hydrolysate so as to separate the chondroitin sulfate and the polyamine.

13. The preparation method according to any one of claims 9 to 12, wherein the raw material comprises an animal tissue, a plant tissue, and a microbial culture fermentation broth.

14. A chondroitin sulfate biogenic polyamine complex prepared and obtained by the preparation method according to any one of claims 8 to 12.

15. Use of a chondroitin sulfate biogenic polyamine complex, wherein the use is any one of the following (a) to (g):
(a) use in the preparation of a drug for anti-inflammation;
(b) use in the preparation of a drug for treating and/or preventing an inflammatory disease;
(c) use in the preparation of a drug for lowering blood lipids;
(d) use in the preparation of a drug for treating and/or preventing hyperlipidemia;
(e) use in the preparation of a drug for treating and/or repairing a joint injury;
(f) use in the preparation of a drug for anti-oxidation; and
(g) use in the preparation of a drug for delaying aging and/or extending a lifespan;
wherein the chondroitin sulfate biogenic polyamine complex is the chondroitin sulfate biogenic polyamine complex according to any one of claims 1 to 7 and 14 and/or a chondroitin sulfate biogenic polyamine complex prepared and obtained by the preparation method according to any one of claims 8 to 13.

16. The use according to claim 15, wherein the inflammatory disease comprises inflammation caused by an inflammation-inducing factor and/or induced by an inflammatory cell, interleukin and/or a tumor necrosis factor, preferably, the inflammatory disease is one or more selected from allergy, eczema, myocardial infarction, cerebral infarction, Alzheimer's disease, dermatitis or arthritis; or
the hyperlipidemia comprises primary hyperlipidemia and/or secondary hyperlipidemia; or
the hyperlipidemia comprises hypertriglyceridemia and/or hypercholesterolemia; or
the hyperlipidemia comprises a hyperlipidemia-associated condition, alternatively, the hyperlipidemia-associated condition comprises a cardiovascular disease, optionally, the cardiovascular disease comprises one or more of arteriosclerosis, coronary artery disease, angina pectoris, carotid artery disease, stroke, cerebral arteriosclerosis, myocardial infarction, cerebral infarction, restenosis after balloon angioplasty, hypertension, intermittent claudication, dyslipidemia, postprandial lipemia, and xanthoma; or
the joint injury comprises a joint injury caused by inflammation, aging, exercise, or injury.

17. Use of the chondroitin sulfate biogenic polyamine complex according to any one of claims 1 to 7 and 14 and/or a chondroitin sulfate biogenic polyamine complex prepared and obtained by the preparation method according to any one of claims 8 to 13 in the preparation of a health food or a cosmetic.

18. A pharmaceutical composition, health product or cosmetic, comprising the chondroitin sulfate biogenic polyamine complex according to any one of claims 1 to 7 and 14 and/or a chondroitin sulfate biogenic polyamine complex prepared and obtained by the preparation method according to any one of claims 8 to 13.
